# EUROPEAN PATENT SPECIFICATION

(11) **EP 2 934 613 B1**
(45) Date of publication and mention of the grant of the patent: **13.02.2019**
(21) Application number: 13818832.1
(22) Date of filing: 20.12.2013
(51) Int. Cl.: A61L 27/54, A61L 29/08, A61L 31/10, A61L 27/28, A61L 15/14, A61L 15/16, A61L 15/44, A61L 27/22, A61L 28/00, A61L 29/16, A61L 31/16, A61L 26/00, G01N 33/543, A61L 27/34

(54) **CLEAVABLE COATING MATERIAL HAVING MICROBIAL FUNCTIONALITY**
SPALTBARES BESCHICHTUNGSMATERIAL MIT MIKROBIELLER FUNKTIONALITÄT
MATÉRIAU DE REVÊTEMENT CLIVABLE AYANT UNE FONCTIONNALITÉ MICROBIENNE

(30) Priority: 21.12.2012 NL 2010040
(43) Date of publication of application: 28.10.2015
(60) Divisional application: 18177306.0
(73) Proprietor: Original G B.V., 9603 AP Hoogezand (NL)
(72) Inventor: GAZENDAM, Jurjen, 9603 AP Hoogezand (NL)
(74) Representative: Nederlandsch Octrooibureau
(86) International application number: PCT/NL2013/050941
(87) International publication number: WO 2014/098603

(56) References cited:
- WO-A2-00/64486
- WO-A2-2008/053362
- US-A- 4 351 337
- US-A- 5 770 229
- WEISSLEDER R ET AL: "Quantitation of slow drug release from an implantable and degradable gentamicin conjugate by in vivo magnetic resonance imaging", ANTIMICROBIAL AGENTS AND CHEMOTHERAPY, AMERICAN SOCIETY FOR MICROBIOLOGY, vol. 39, no. 4, 1 April 1995 (1995-04-01), pages 839-845, XP002114983, ISSN: 0066-4804

## Description

The present invention relates to a an object comprising a polymer coating, the coating comprising (a) one or more polymers, said polymers comprising a first cleavage site, and (b) a first agent releasable from said coating upon cleavage of said first cleavage site. The invention further relates to a method for coating an object and to such a polymer coating.

In the art, objects of the above type are known from e.g. US8372420 wherein an object is described, in particular an implant, coated with a coating matrix comprising releasable antimicrobial agents for the treatment of infection on an implant. The agent is released from the matrix as a result of pH decrease in the host, e.g. as a result of a bacterial infection at the location of the implant. By the release of the antimicrobial agents, an infection at the location of the implant can be treated. In this way, invasive surgery to treat an infected implant can be avoided. However, a change in pH is the result of any unspecific bacterial infection. Further, according to US8372420, a microbial infection at the site of the implant cannot be detected as such. There is merely a release of an antimicrobial agent in an unspecific manner without any signalling regarding the detection of a microbial infection. The said agents can be bound to the said coating matrix in any manner, such as covalently and non-covalently. As one out of many examples, the possibility of a covalent bonding of the agent to the polymer coat via a linker, cleavable by an endogenous enzyme, expressed by the host upon bacterial infection, is disclosed.

US6306422 describes an implant coated with a hydrogel matrix, comprising embedded releasable active agents, such as therapeutic and/or diagnostic agents. By a pH change in the host at the location of the implant, which pH change is induced by a bacterial infection, the hydrogel matrix swells by uptake of water that is present in the vicinity of the hydrogel, and the embedded active agents are released. As diagnostic agent dyes are described, that visualize the site of infection. This hydrogel matrix described by US6306422 does not respond to a specific microbe, but like that of US8372420 to a general, environmental change at the site of the implant.

US4351337 describes an implant comprising matrix coat material for slow release of active agents, including therapeutic agents and diagnostic agents such as a dye to monitor the presence or absence of a pathological condition. The implant matrix is not sensitive to microbial infection, but to enzymes present in the host carrying such an implant. These enzymes produced by the host act as a general response to certain pathological conditions, and are also not released upon a specific microbial infection, but on a number of more general conditions in the host. The release of the agents is hence only possible after a response of the host to a pathological condition.

WO2008/053362 describes implantable polymeric objects, comprising, in the primary platform thereof, a polymer comprising a biologically active agent covalently attached to the polymer via a polyamide linker susceptible to selective hydrolysis by peptidase enzymes. Upon hydrolysis, the active agent is released from the polymer.

US5770229 describes an implant comprising a medical polymer gel comprising a cleavable group, cleaved by an enzymatic reaction and a drug, such as the antibiotic gentamycin. The cleavable group can be cleaved by an enzyme, produced by bacteria, so that the antibiotic is released from the implant when implanted at an infection site. A similar implant is described in WO00/64486.

So, in the art, objects prone to infection having a coating comprising an agent reactive to antimicrobial infection are known. However, such agents are limited to antimicrobial agents, released from the coating as a result of a general antimicrobial stimulus. The release of an agent from the coating is triggered by a conditional change, e.g in temperature or pH, which change is not related to infection by a specific micro-organism, and does even not need to be related to an infection caused by microbes, but merely a sterile infection or a general inflammatory response. The coating will respond to any change in environmental factors, for example any infection, that is, the response is not specific. Further, polymeric implants are known, wherein the polymeric matrix comprises cleavable active agents. Such implants however do not have a coat comprising an agent reactive to antimicrobial infection, but comprise the polymer in their matrix, i.e. in their primary platform, which makes sense, as these implants are designed to provide an active agent in a sustained release fashion over longer periods of time.

The present inventors have now provided an object, i.e.implants, comprising a coating that releases an agent upon infection by a specific microbe, or a specific group of microbes, consisting of a Gram positive bacteria. The release of that agent is indicative for the presence of such a microbe. The present invention thereto provides an object comprising a polymer coating, the coating comprising
a) one or more polymers, said polymers comprising a first cleavage site and
b) a first agent releasable from said coating upon cleavage of said first cleavage site,
characterized in that the first agent is covalently bound to a first polymer comprising polyethylene glycol via a first linker, the first linker comprising the first cleavage site being a peptide,
wherein the first cleavage site is cleaved by a sortase enzyme specifically provided by a microbe belonging to a first group consisting of a Gram positive bacteria, and not cleaved by any compound provided by any microbe not belonging to said first group, wherein cleavage of the said first cleavage site results in release of the said first agent from the coating, the release of said first agent being indicative for the presence of a microbe belonging to the said first group, wherein the first agent is a diagnostic agent being a fluorescent agent.

The novel objects release an agent from the polymeric coating thereof when a cleavage site, present on the polymer, is cleaved. This cleavage is effected by a sortase enzyme, provided by a microbe, belonging to a group consisting of Gram positive bacteria. Members of this group, as a matter of ease indicated as the first group, are capable of specifically providing a compound being a sortase enzyme, that cleaves the said first cleavage site. The term 'specifically' intends to mean that microbial strains, species or genera not belonging to the said first group are not capable of providing a compound that can recognize and cleave the said cleavage site, in contrast to members of said first group. The compound can be provided e.g. by release of the said compound from a microbe, or can e.g. be presented on the outer surface thereof. In the latter case, cleavage will be effected upon contact of the microbe with the coating, whereas when the compound is released from the microbe, the microbe can be at a distance of the coated object, as long as the released compound is capable to arrive at the coating after release from the microbe. . For microbes to belong to a certain group, such as Gram positive bacteria, the presence of these microbes must induce cleavage of the cleavage site, whereas cleavage is not induced by microbes from another group. This cleavage can occur by the same compound, but also by a plurality of different compounds, as long as these compounds induce cleavage of the said same cleavage site. For example, the first group comprises three different bacterial species, the first and second species providing the same compound cleaving the cleavage site of the coating, the third species providing a compound, differing from the compound from the first and second species, but also specifically cleaving the same cleavage site in the coating.

Release from the first agent is effected upon cleavage of the first cleavage site by the sortase enzyme.

The release of the first agent is indicative for the presence of one or more microbes belonging to the said first group, as only microbes belonging to the said first group are capable of providing a sortase enzyme, capable of cleaving the said first cleavage site. The first agent can therefore be only released from the coat when the first cleavage site is cleaved. For that cleavage, a member of the first group of Gram positive bacteria must be present. So observation of released first agent is indicative for the presence of such a microbe.

The group of Gram positive bacteria can comprise a single microbial strain, species or genus, or a limited number, i.e. not all different species from a genus. The first group can also encompass Gram-positive bacteria, excluding Gram-negative bacteria, or a limited number of such Gram positive bacteria.

Sortase is an example of a class of compounds that is produced by Gram-positive bacteria. Gram-positive bacteria is a group of bacterial genera that can be further defined by the presence of a peptidoglycan comprising cell wall and representative examples of Gram-positive bacteria include clinically relevant microbial genera such as *Staphylococcus, Streptococcus, Enterococcus* (cocci), *Bacillus, Clostridium* and *Listeria.* All genera belonging to the group of Gram-positive bacteria produce a so-called sortase enzyme, recognizing and cleaving a cleavage motif in peptides and proteins. When the cleavage site of the polymer of the coating contains a motif recognized by such a sortase, the agent can be released from the coating in the presence of one or more of the corresponding Gram-positive bacteria. Release of the said agent is therefore indicative for the presence of said Gram-positive bacteria, which is further elucidated below.

As can be seen from the explanation of the invention given above, the group consisting of Gram positive bacteria may consist of only a single member, in the case of a strain specific compound, , or may contain several members such as the group of Gram positive that contains at least six clinically relevant genera for example *Staphylococcus, Streptococcus, Enterococcus* (cocci), *Bacillus, Clostridium* and *Listeria.*

The term 'microbe' is well understood in the art, and comprises single and multicellular organisms, such bacteria, fungi, protozoa, viruses and bacteriophages, carrying their genome necessary for multiplication. Multicellular organisms are regarded as microbes, as long as their cells are not differentiated. In the present application the term microbes relates in particular to pathogenic bacteria. Examples of genera of pathogenic bacteria include, but are not limited to, *Campylobacter, Chlamydia and Chlamydophila, Clostridium, Cornynebacterium, Dermabacter, Enterococcus, Escherichia, Granulicatella, Helicobacter, Legionella, Mycobacterium, Neisseria, Pseduomonas, Salmonella, Staphylococcus, Streptococcus* and *Vibrio.*

A further advantage of the present invention is that the sortase enzymes provided by a group of Gram positive bacteria need not be exactly the same, as long as these compounds cleave the said cleavage site. Many compounds produced by microbes have similar but not the same molecular structure and are thus known in the art as being homologous. Homologous compounds may contain the same recognition site for a cleavage site, but the molecular structure distant from the recognition site is different. Examples of homologous compounds are the compounds produced by *Vibrio cholera* strains CHA6.8ΔprtV and VC1649 respectively, which both cleave the same motif but which compounds have different lengths. The benefit of recognizing a specific motif rather than the entire compound is that if there is a spontaneous mutation in the structure of the compound, the compound will still cleave the cleavage site and thus the agent will still be released.

Microbial strains, species or genera that do not produce such a compound capable of cleaving the first cleavage site do not therefore belong to the said first group. In this way it is possible to prevent the agent being released from the polymer coating in the presence of, for example, healthy gut flora microbes but the agent will be released in the presence of pathogenic bacteria, which secrete a compound capable of cleaving the first cleavage site.

Interaction of such a compound produced or provided by microbes belonging to said first group with the said first cleavage site will result in cleavage and therefore release of the agent. Release of the agent is therefore indicative for the presence of a microbe belonging to the said first group. This is different from the state of the art because the cleavage in the state of the art is facilitated by a general response to microbes, for example a change the pH or temperature surrounding the object and not to a specific microbial product.

The agent released from the coating can be a signaling molecule that gives a detectable signal upon release from the coating. The agent therefore is only detectable in the presence of a member of a group of microbial strains, species or genera. Such signaling molecules are known in the art. And discussed further below.

The agent may also be a therapeutic agent, for example an antibiotic. In this instance, the presence of an infection cannot be detected visually, but by the presence of released antibiotic. The antibiotic further provides a preventative measure to prevent an infection from breaking out. Any suitable antibiotic that can be bound to the polymer coating may be used, but the antibiotic can be chosen to match the type of microbial strain, species or genus that will cause cleavage of the agent. For example, if the cleavage site is specific for Gram-positive bacteria then a broad spectrum, Gram-positive specific, antibiotic such as Vancomycin can be used as the agent. The ability to tune the antibiotic to suit the cleavage site is a further advantage of the present invention over the prior art as in the polymer coatings of the prior art it was not possible to specifically tune the antibiotic and cleavage site to a specific group of microbial strain, species or genus.

The release of the agent, be it a diagnostic or therapeutic agent, is therefore indicative for the presence of a Gram positive bacteria.

The agent may be covalently bound to the polymer coating by a linker, which linker comprises the cleavage site.

The polymer coating according to the present invention can be coated onto an object whereby the term 'polymer coating' is understood to mean a composition comprising a polymer that at least partially covers and adheres to the said object. The object to be coated can be any object for which detecting and or treating the presence of microbes on said object is necessary.

Although the present invention also encompass medical implants solely intended for release of an agent there from, i.e. implants intended as e.g. sustained release vehicles, in a preferred embodiment of the invention the object has at least a primary first function and a second auxiliary function, the second auxiliary function being the release of at least the first agent from the coating, the first primary function being unrelated to the said coating or the release of the agent therefrom. In a medical implant solely intended for release of the agent there from, the first and probably sole primary function is the release of the agent from the implant.

However medical apparatus and medical implants, usually are implanted for a certain medical reason, such as a hip prosthesis for example, or an infusion needle. The primary function is therefore to provide hip replacement function, or entrance for medicinal liquids in the blood stream of a patient, respectively. Such objects need to remain free from infection and the present invention provides a simple way in which to detect and or treat the presence of microbes on said apparatus and implants. The release of the agent, therapeutic or diagnostic or both, is a secondary function of such an object, not related to the primary function. The invention is particularly suited to objects prone to biofilm formation, for example orthopedic plates and pins. The object to be coated is therefore chosen to perform a function, such as reinforcing a bone in a patient, and the polymer coating adheres to the object so that the object can, while performing its intended primary function, also detect or treat the presence of Gram positive bacteria via the unique reactivity of the cleavage site contained in the polymer coating. The present invention therefore has the advantage that the polymer coating comprising a cleavage site specific for Gram positive bacteria, can be applied to a wide range of clinical apparatus using coating techniques well known to the skilled person.

Many microbes use a number of secretory systems to secrete one or more compounds which enable the microbes to invade the host organism, such a mammal, e.g. a human. Such secreted compounds are often enzymes and such enzymes are highly specific for certain molecular motifs. The secreted enzymes may be membrane bound and thus remain close to the surface of the microbial cell wall, or be secreted into the surrounding medium. The inventors have therefore used this specificity in an inventive manner by incorporating a cleavage site that is only cleaved by an enzyme produced by a Gram positive bacteria into a polymer coating.

An enzyme secreted by a pathogenic bacterium that can be chosen as a compound to cleave a cleavage site according to the invention is sortase, as already discussed above. The word sortase as used in this application refers to the generic class of sortase proteases. Sortases have been classified into four groups (sortase classes A-D) on the basis of sequence homology, the substrate for sortase cleavage and the nucleophile accepted by the sortase (see Antonio, Nature Rev Microbiology 2011, 9, 166-176; Microbiology and Molecular Biology Reviews, 2006, 192-221) Sortase is a protease secreted by, for example, *Staphylococcus aureus.* The function of sortase is to connect proteins on the surface of *S. aureus* with host proteins in order to evade host defence mechanisms. As an exemplary sortase, sortase A is responsible for the anchoring of 20 different surface proteins to the cell wall of *S. aureus* strain. One of these surface proteins, protein A, binds to the Fc terminus of mammalian immunoglobulins in a nonimmune fashion, causing decoration of the staphylococcal surface with antibody. By this mechanism *S. aureus* can evade the human immune system.

Sortase class A enzymes are present in all Gram-positive bacteria and are often referred to as housekeeping sortases. A cleavage site that is recognised by sortase A can therefore be incorporated into the present invention in cases where the risk of infection from any Gram positive bacterium is high, so that the agent can be released from the polymer coating in the presence of any Gram positive bacterium.

The product of the sortase class A reaction is a surface protein that is covalently linked to lipid II and is then incorporated into the cell wall envelope. Sortase class A substrates include surface proteins from the microbial surface components recognizing adhesive matrix molecules (MSCRAMM (SEQ NO: 1)) family that have been implicated in the virulence of multiple Gram-positive bacterial species.

Sortase class B enzymes are encoded by a limited number of microbial species, including *Staphylococcus spp., Bacillus spp., Listeria spp.* and *Clostridium spp.* Sortase class B enzymes recognize a unique NP(Q/K)TN sorting signal in proteins that are involved in haem-iron scavenging, and cross-link the anchored haem-containing products near membrane transporters. The β-barrel structure of sortase class B enzymes is similar to that of the class A enzymes but encompasses additional α-helices.

An example of a compound that recognises the same cleavage site as Sortase A B but has a different structure is the pilin-specific class C sortases. It is therefore possible that if sortase A is not produced in sufficient quantity to cleave the sortase A cleavage site, the pilin-specific class C sortase will cleave the said cleavage site. Such multiple recognition of the same cleavage site by different compounds produced by the same microbial species increases the likelihood of the agent being released in the presence of specific, or a limited number of, microbial strains, species or genera. Without wishing to be bound by theory, in contrast to sortase A that only accept single cell wall peptides as nucleophiles, pilin-specific sortases recognize multiple pilin proteins both as substrates for and as nucleophiles. Pilin-specific class C sortases are encoded in pilin gene clusters with their cognate substrates and often contain a C-terminal hydrophobic domain not found in sortases from other classes. The crystal structures of the three pilin-specific sortase class C enzymes from *Streptococcus pneumoniae* (SrtB, SrtC and SrtD (also known as SrtC1, SrtC2 and SrtC3, respectively)) provide mechanistic insights at a molecular level so that the cleavage site can be designed to ensure optimal cleavage by the said enzymes.

A further example where cleavage is facilitated by a compound provided by a limited number microbial species is taken from the group of spore forming microorganisms, containing for example *Bacillus spp.* and *Streptomyces spp.*. The said spore forming microorganisms provide a compound called sortase D. When the first cleavage site contains a recognition motif for sortase D, the first agent will be released only in the presence of a spore forming microorganism. In other words, the release of an agent from a polymer coating wherein the polymer coating comprises a first cleavage site cleaved by sortase D, the said release is indicative for the presence of a spore forming microorganism. Regarding the chemical structure of sortase D, no crystal structure of this enzyme has been solved.

The first agent is covalently bound to a first polymer via a first linker, the first linker comprising the first cleavage site. It is however also possible for the first agent to be bound to a one or more different polymers. With 'different polymers' is meant, unless indicated otherwise, to relate to different types of polymers, not to different molecules of the same polymer.

A cleavage site is hereby understood to have its well known meaning, that is a portion of molecular structure of the coating, as an intrinsic part thereof or adhered thereto, that is cleaved by a specific compound. The cleavage sites discussed herein are, in an embodiment of the invention, are suitable to be incorporated in a linker between the polymer coating and the first agent so that it is possible to release the first agent selectively in the presence of a microbe that secretes a compound specific for said first cleavage site.

The first linker preferably comprises one or more bonds chosen from the group, consisting of amide bonds, ester bonds, thioester bonds, carbamate bonds, urethane bonds, the first linker preferably comprising amide bonds and most preferably a peptide.

In an example where the linker comprises amide bonds, the linker may have the structure XYZ, wherein X is at least one amino acid, Y is a portion of molecular structure composed of at least three amino acids representing the first cleavage site, and Z is at least one amino acid. The amino acids used may be any amino acid, preferably α amino acid, preferably chosen from the group of naturally occurring amino acids orfrom the group of synthetic amino acids, in particular derivatives of natural amino acids. The peptide linker can comprise for example between 4 and 10, for example amino acids, for example between 5 and 11 amino acids, for example between 6 and 12 amino acids, for example between 7 and 13 amino acids and for example between 8 and 14 amino acids.

A cleavage site preferably comprises of a number of amino acids, for example at least two, preferably at least three amino acids, more preferably at least four amino acids, even more preferably at least five amino acids, as described in the standard handbook Biochemistry, Ed. L Stryer, 7th Edition, 2012, WH Freeman, Part 1, Chapter 2 Protein Composition and Structure. The portion of molecular structure in a cleavage site may also be referred to as an 'amino acid motif.' In the present invention, the cleavage site is designed such that it is recognized by a compound provided by a microbe belonging to a group consisting of a Gram positive bacteria.

Examples of suitable linkers can be cleaved by, for example, a thrombin type protease could be a linker comprising for example 14 amino acids: GGGGfPRGFPAGGG (SEQ NO: 13) whereby X is 3 amino acids and represents part of the linker, Y is 8 amino acids and represents the first cleavage site (underlined amino acids) and Z is 3 amino acids and represents part of the linker. The standard amino acid notation is used throughout, whereby a each amino acid is denoted by a single letter. A capital letter denotes an "L" configured amino acid and a lower case amino acid denotes a "D" amino acid, as described in Stryer, *supra,* Chapter 24.

In another embodiment, the at least first cleavage site comprises a specific amino acid motif, preferably being chosen from the group, consisting of LPATG (SEQ NO: 4), LPETG (SEQ NO:5), LPDTG (SEQ NO:6), LPQTG (SEQ NO: 7), NPQTN (SEQ NO: 8), NPKTN (SEQ NO: 9),

As described above, the cleavage site of the polymer comprises a portion of molecular structure that is recognised by a compound produced by a limited number of microbial strains, species or genera. The term 'specific is herein used to describe an amino acid motif that is only recognised by a particular compound produced by a microbe belonging to a first group consisting of a Gram positive bactera. The cleavage site may comprise a number of amino acids or other molecules such as heterocyles or lactones or unsaturated aliphatic carbon bonds that can be recognised by a compound provided by said microbe.

A further example of a specific amino acid motif is provided by the Sortase class of enzymes. Sortase class A recognize the amino acid motif LPXTG at the carboxyl terminus of surface protein precursors, where X can be any amino acid. Sortase class B enzymes recognize a unique NP(Q/K)TN (SEQ NO: 8 / SEQ NO: 9), while the pillin specific sortases cleave the LPXTG-like motif.

Without wishing to be bound by theory, the specificity of cleavage is determined by recognition of sortase-specific motifs (LPXTG or NP(Q/K)TN). Each sortase cleaves its specific motif and subsequently forms an acyl (thioester) bond between the sortase active site and a residue in the sorting signal. This acyl intermediate is then resolved by a specific nucleophilic amino acid, thus specifying the cell wall component to which the protein becomes linked. For example, in *Staphylococcus aureus* the class A sortase cleaves the LPXTG sorting signal between the Thr and Gly residues to form an acyl intermediate between the Thr residue of the surface protein and a reactive Cys in the TLXTC (SEQ NO:12) catalytic pocket of the sortase. Subsequently, the acyl intermediate is resolved by nucleophilic attack by the cell wall precursor lipid II.

In particular, by means of a non-limiting example, *Staphylococcus aureus,* a pathogenic bacterium, secretes SrtA. which cleaves the pentapeptide motif LPETG (SEQ NO:5) and thus the LPETG (SEQ NO:5) motif can be used as a cleavage site specific to *Staphylococcus aureus.* A further example of a pentapetide motif cleaved by *S. aureus* is LPATG (SEQ NO:4).

A reference handbook which the skilled person can use in order to find data regarding the substrates for proteases Handbook of Proteolytic Enzymes, 2nd - Editor(s) : Barrett & Rawlings & Woessner, 2004.

As described above, the first agent is covalently bound to the polymer via a linker, which linker comprises the first cleavage site. The linker and cleavage site preferably consist of a number of amino acids, in other words, the linker and cleavage site comprise a peptide.

A peptide comprising the linker and the cleavage site can be synthesized via solid phase peptide synthesis. The person skilled in the art is able to synthesize the desired sequence using commercially available building blocks and reagents described in Merrifield ,1973, Chem. Polypeptides, 335-361 (Katsoyannis and Panayotis eds.); Merrifield, 1963, J. Am. Chem. Soc., 85:2149; Davis et al.,1985, Biochem. Intl., 10, 394-414; Stewart and Young, 1969, Solid Phase Peptide Synthesis; U.S. Pat. No. 3,941,763; Finn et al., 1976, The Proteins (3rd ed.) 2, 105-253; and Erickson et al., 1976, The Proteins (3rd ed.) 2, 257-527. The use of protecting groups, linkers, and solid phase supports, as well as specific protection and deprotection reaction conditions, linker cleavage conditions, use of scavengers, and other aspects of solid phase peptide synthesis are well known and are also described in "Protecting Groups in Organic Synthesis," 3rd Edition, T. W. Greene and P. G. M. Wuts, Eds., John Wiley & Sons, Inc., 1999; NovaBiochem Catalog, 2000; "Synthetic Peptides, A User's Guide," G. A. Grant, Ed., W.H. Freeman & Company, New York, N.Y., 1992; "Advanced Chemtech Handbook of Combinatorial & Solid Phase Organic Chemistry," W. D. Bennet, J. W. Christensen, L. K. Hamaker, M. L. Peterson, M. R. Rhodes, and H. H. Saneii, Eds., 1998, Advanced Chemtech; "Principles of Peptide Synthesis, 2nd ed.," M. Bodanszky, Ed., Springer-Verlag, 1993; "The Practice of Peptide Synthesis, 2nd ed.," M. Bodanszky and A. Bodanszky, Eds., Springer-Verlag, 1994; "Protecting Groups," P. J. Kocienski, Ed., Georg Thieme Verlag, Stuttgart, Germany, 1994.

Briefly, two types of solid phase peptide synthesis are known in the art, referred to as Fmoc and Boc solid phase peptide synthesis respectively. It is well known in the art that Fmoc peptide synthesis can be implemented more readily than Boc peptide synthesis, and the techniques necessary to carry out Fmoc solid phase peptide because Fmoc synthesis does not require specialized operating procedures to contain the extremely reactive acids used. Fmoc synthesis is described in the Handbook: Fmoc Solid Phase Peptide Synthesis: A Practical Approach (Practical Approach Series), Chan and White, Oxford University Press, 1999.

The thus synthesized cleavage site can be coupled to the polymer coating on the one hand and the first agent and / or other additional agents on the other hand using standard coupling reactions known to the skilled person. Such methods are described in "Methods of preparing peptide-carrier conjugates" by Ian W. Drifthout and Peter Hoogerhout in the Handbook: Fmoc Solid Phase Peptide Synthesis: A Practical Approach (Practical Approach Series), Chan and White, Oxford University Press, 1999. Further techniques on crosslinking a polymer with a protease cleavage site is contained in the Thermo Fischer Crosslinking Handbook.

The functionalities for crosslinking may be all the same or combinations of functionalities, and may include the functionalities of naturally occurring amino acids, such as amino, e.g. lysine, carboxyl, e.g. aspartate and glutamate, guanidine, e.g. arginine, hydroxyl, e.g. serine and threonine, and thiol, e.g. cysteine. Preferably, the functionality contains nitrogen, for example amino (NH₂), for example guanidine (NHCNHNH₂).

The crosslinking agent will normally be difunctional, where the functionalities may be the same or different, although higher functionality may be present, usually not exceeding four functionalities (Stedronsky US 6,423,333). Depending upon the particular functionalities available on the polymers, various crosslinking agents may be employed. The crosslinking agents will usually be at least about three carbon atoms and not more than about 50 carbon atoms, generally ranging from about 3 to 30 carbon atoms, more usually from about 3 to 16 carbon atoms. The chain joining the two functionalities will be at least one atom and not more than about 100 atoms, usually not more than about 60 atoms, preferably not more than about 40 atoms, particularly not more than about 20 atoms, where the atoms may be carbon, oxygen, nitrogen; sulfur, phosphorous, or the like. The linking group may be aliphatically saturated or unsaturated, preferably aliphatic, and may include such functionalities as oxy, ester, amide, thioether, amino, and phosphorous ester. The crosslinking group may be hydrophobic or hydrophilic.

Various reactive functionalities may be employed, such as aldehyde, isocyanate, mixed carboxylic acid anhydride, e.g. ethoxycarbonyl anhydride, activated olefin, activated halo, amino, and the like. By appropriate choice of the functionalities on the peptide linker and polymer coating, the crosslinking agent, rate of reaction and degree of crosslinking can be controlled.

Various crosslinking agents may be employed. Crosslinking agents which may be used include dialdehydes, such as glutaraldehyde, activated diolefins, diisocyanates such as, tetramethylene diisocyanate, hexamethylene diisocyanate, octamethylene diisocyanate, acid anhydrides, such as succinic acid dianhydride, ethylene diamine tetraacetic acid dianhydride, diamines, such as hexamethylene diamine, cyclo(L-lysyl-L-lysine), etc. The crosslinking agent may also contain unsymmetrical functionalities, for example, activated olefin aldehydes, e.g. acrolein and quinoid aldehydes, activated halocarboxylic acid anhydride, and the like. The crosslinking agents will usually be commercially available or may be readily synthesized in accordance with conventional ways, either prior to application of the adhesive or by synthesis in situ.

Usually, the polymer coating will be available as a dispersion or solution, particularly aqueous, generally the concentration of the protein polymer being in the range of about 50 mg to 1 g/ml, more usually from about 100 to 800 mg/ml. The solution may be buffered at a pH which enhances or retards the rate of crosslinking. Usually the pH will be in the range of about 2 to 12, more usually 8 to 11. Various buffers may be used, such as phosphate, borate, carbonate, etc. The cation can have an effect on the nature of the product, and to that extent, the alkali metals potassium and sodium, are preferred. The protein composition will generally be about 5 to 40, more usually from about 5 to 20, preferably from about 10 to 20 weight %, to provide for a composition which may be readily handled, will set up within the desired time limit, and the like. The buffer concentration will generally be in the range of about 50 to 500 mM. Other agents may be present in the protein solution, such as stabilizers, surfactants, and the like. If the polyfunctional second compound is present its concentration will be determined in accordance with its ratio to the crosslinking agent and the polymer.

The ratio of crosslinking agent to polymer will vary widely, depending upon the crosslinking agent, the number of functionalities present on the polymer, the desired rate of curing, and the like. Generally, the weight ratio of polymer to crosslinking agent will be at least about 1:1 and not greater than about 100:1, usually not greater than about 50:1, generally being in the range of about 2 to 50:1, but in some instances may not be more than 30:1. The equivalent ratio of protein to crosslinking agent will generally be in the range of about 0.1-1:3, more usually in the range of about 0.5-2:2. Considerations in selecting the polymer - peptide-crosslinking agent equivalent ratio will be the rate of setup, reactivity of the crosslinking agent, relative solubility of the crosslinking agent in the mixture, physiological properties of the crosslinking agent, desired degree of stability of the crosslinked product, and the like.

Different synthetic routes are possible to prepare polymer -peptide conjugates. For the convergent synthesis of peptide-synthetic polymer conjugates, either via selective modification of a suitable end reactive polymer or via functionalization of appropriate reactive side chains, a broad range of reactions is available.

Traditional peptide coupling conditions are preferably used for the synthesis of peptide- polymer coating conjugates. These reaction conditions are attractive as they generally provide high conversions and are compatible with standard solid phase peptide synthesis protocols. N-terminal modification of solid-supported peptides with carboxylic acid-modified poly(ethylene glycol) (PEG) derivatives, for example, has been widely used to prepare a variety of peptide-synthetic polymer conjugates. A drawback is that these reactions are usually not bio-orthogonal and cannot be used to synthesize conjugates in homogeneous (aqueous) solution from unprotected peptides.

There is, however, a plethora of bio-orthogonal coupling reactions that can be used for this purpose. Examples include various Pd(0)-catalyzed coupling reactions, Staudinger ligation, cycloaddition reactions (Diels-Alder, 1,3-dipolar cycloaddition), reductive alkylation, oxime and hydrazine formation, thiol addition reactions, and oxidative coupling. Although various of these strategies have been used to prepare protein-synthetic polymer conjugates (vide infra), only the Huisgen 1,3-dipolar cycloaddition reaction has been employed in several instances for the synthesis of peptide-synthetic polymer conjugates. It is obvious that the range of other bio-orthogonal coupling reactions that is available provides ample opportunities for the synthesis of novel, complex peptide-synthetic polymer conjugates without the need for possible complex protective group strategies.

The divergent synthesis of peptide-synthetic polymer conjugates can be carried out on the solid phase as well as in homogeneous solution. Divergent solid phase synthesis of peptide-synthetic polymer conjugates is frequently carried out using commercially available Tentagel resins in which poly(ethylene glycol) chains are attached to the solid support via a labile linker. To avoid aggregation and facilitate the synthesis of difficult peptide sequences, so-called switch ester segments can be introduced in the peptide backbone. After completion of the synthesis, the peptide backbone can be reestablished via a selective O to N acyl switch. In addition to the use of switch ester segments, there are various other strategies that can be used to prepare difficult peptide sequences that are prone to aggregation including the use of backbone amide protecting groups or microwave synthesis. Instead of grafting the peptide segment step-by-step from a soluble or insoluble synthetic polymer support, the synthetic polymer segment can also be grown from appropriate initiator modified resin-bound peptides using controlled radical polymerization techniques.

These polymerization techniques have also been used to synthesize peptide-synthetic polymer conjugates in homogeneous solution. Using peptide-functionalized chain transfer agents (CTA's), for example, peptide-synthetic polymer conjugates can be prepared via reversible addition-fragmentation chain transfer (RAFT) polymerization. While the peptides may be attached to either the Z or the R group of the CTA, the latter approach is particularly attractive as it can be used to generate thiol end-functionalized peptide-synthetic polymer conjugates. The thiol end group represents a versatile handle for further bio-orthogonal chain end functionalization and can also be used to coat gold substrates with a monolayer of the conjugates. Several reports have described the synthesis of peptide-synthetic polymer conjugates using cyclic or linear peptide atom transfer radical polymerisation (ATRP) initiators. These ATRP initiators were typically obtained by modification of the N- or C-terminal amino acid or a suitable side-chain functional group with the appropriate ATRP initiator. Recently, Maynard et al. (2008, J. Am. Chem. Soc., 1041-1047) have taken this concept a step further and synthesized artificial amino acids with side-chain ATRP initiators that are compatible with standard Fmoc SPPS. With these amino acids it is now possible to synthesize peptide initiators with exact control over the conjugation site without having to rely on the occurrence of a particular amino acid in that peptide or the use of elaborate protective group strategies.

A final, divergent approach for the synthesis of peptide-synthetic polymer coating conjugates is based on the polymerization of peptide-based monomers. A variety of peptide-based monomers have been polymerized via conventional free radical polymerization, ring-opening metathesis polymerization, or controlled radical polymerization.

The polymer coating may be a biodegradable or non-biodegradable polymer coating. Any polymer coating may be suitable for use in the present invention as a coating on an implant provided that the polymer coating can be engineered to contain a suitable protease cleavage site. For example, the polymer coating can be engineered such, that it contains a specific cleavage site, susceptible to cleavage byGram positive bacteria, within the polymer coating itself, and the therapeutic or diagnostic agent entrapped within the polymer coating is thereby released.

The cleavage site may comprise the said pentapeptide LPTEG motif from *S. auerus* between the polymer coating and the at least first agent and or the at least second agent. Preferably the cleavage site comprises the sequence PPTP (SEQ NO:2), PPSP (SEQ NO: 3).

The first agent is a diagnostic agent.

The first agent is a diagnostic molecule, the release of the first agent is therefore indicative for the presence of a particular microbial strain, species or genera. The presence of released diagnostic agent is indicative for the presence of the corresponding microbes.

The advantage of the first agent being a diagnostic agent is that a facile way is provided to determine whether an object, for example, a medical implant is infected by a microbial strain, species or genus, without the necessarily resorting to a surgical biopsy to sample the tissue surrounding the medical implant.

In a the present invention, when the object is to be implanted in a host, such as a mammalian host, the first agent is preferably a photon emitting agent being a fluorescent, agent, wherein the fluorescent agent preferably is IRDye®800CW. Such agents are detectable using an optical imaging technique such as near-infrared imaging. Optical imaging within the near infrared (NIR) window (650-900 nm) is advantageous, because tissue autofluorescence is very low and the penetration of this longer-wavelength light into tissues is significantly better than in other regions of the spectrum. A preferred agent is IRDye™ 800CW (excitation / emission = 774/789 nm) which emits directly in the "sweet spot" of the NIR window, where contributions from unwanted sources are minimal, so it has the potential to be an effective beacon to highlight the location of microbial infection. Upon bacterial infection by a specific microbe, the said microbe will secrete a sortase enzyme that specifically cleaves the cleavage site contained in the polymer coating, resulting in release of the said first agent. If the first agent is a dye, for example, IRDye®800CW, use of an near-infrared imaging unit provides an efficient way provided to detect an infection caused by a limited number of microbial strains, species or genera. Near-infrared imaging units are frequently used in clinics and therefore the present invention is applicable to current industry practices.

In biological applications the use of fluorescent agents is preferable for reasons of general applicability, toxicity and synthesis. The said photon emitting agent should preferably emit upon release from the material, but not emit, or at least emit significantly less, if still entrapped in or bound by the said material.

The skilled person can apply the standard teachings from Handbook of Photonics for Biomedical Science Editor: Valery V. Tuchin, 2010, which describes important methods, such as finite-difference time-domain simulation, for the mathematical modeling of light transport and interaction with cells, discusses optical and terahertz spectroscopy and imaging methods for biomedical diagnostics, presents novel modalities of photon ballistic, multidimensional fluorescence, Raman, confocal, CARS, and other nonlinear spectroscopies that provide molecular-level cell and tissue imaging, explores key photonic technologies for therapy and surgery, including photodynamic, low-intensity laser, and photothermal therapies, explains how nanoparticle photonic technologies are used for cancer treatment and human organism protection from UV radiation, focuses on advanced spectroscopy and imaging of a variety of normal and pathological tissues, such as embryonic, eye, skin, brain, and gastric tissues.

In a further embodiment, the object comprises a first additional agent, which is released upon cleavage of the first cleavage site. In addition to the first agent, now also the first additional agent will be selectively released when the first cleavage site is cleaved.

The said first agent is a diagnostic agent, and the first additional agent is a therapeutic agent. This means that upon infection of the coating with a microbe of the envisaged group, both the first agent and the first auxiliary agent will be released. It is advantageous to chose for a diagnostic agent (such as an above-described photon emitting agent) as first agent and for a therapeutic agent (such as an antibiotic) as first additional agent.. By such a combination, both the infection can be easily visualised whereas the infection is simultaneously attacked by the antibiotic.

In an attractive embodiment, the first additional agent is covalently bound to a first additional polymer via a first additional linker, the first additional linker comprising the first cleavage site. The invention also allows for the first and first additional agents to be linked to different linkers. A polymer comprising a linker may be prepared, where the linker is bound to the first agent via a cleavage site, wherein the linker comprises, in general terms, XY aminio acids, where X represents the amino acids of the cleavage site. The total supply of polymer-linker construct can split into two (or more) reaction vessels enabling a fist portion of this polymer- linker construct to be covalently bound to the first agent via the Y amino acid. Another portion of the polymer-linker construct in the second vessel may be bound to the first additional agent via the Y amino acid. The two polymer-linker constructs comprising either the first agent or the first additional agent can then be combined and used to coat an object. Both constructs comprise different linker molecules having the same cleavage site (composed of X amino acids) and therefore both the first and first additional agents will be released in the when the cleavage site is cleaved by a sortase enzyme produced by a Gram positive bacteria, wherein said compound is specific for said cleavage site. It is also possible to link both a first agent and a first additional agent to the linker molecules in a single step in a single vessel.

As outlined above, it is possible to provide for two different diagnostic agents or a diagnostic agent and a therapeutic agent, or two different therapeutic agents contained within the polymer coating of the object to of the present invention, and released by cleavage of the same cleavage site.

It is therefore possible to construct a polymer coating that on cleavage of the first cleavage site, a diagnostic agent and a therapeutic agent are released, which diagnostic agent is indicative of a particular microbial strain, species or genus and the therapeutic agent targets the said particular microbial strain, species or genus.

It is also possible to prepare a first polymer with the first agent coupled thereto via the first linker, and a first additional polymer, with the first additional agent coupled thereto with the same linker, or with a different linker but comprising the same cleavage site, or at least a site that is cleaved by the same compound as the first cleavage site. Preferably, the first additional linker is identical to the first linker.

In another attractive embodiment, the first additional polymer is identical to the first polymer. Using the same polymer for binding both the first and first additional agent enables the preparation of a uniform and homogenous coating. It is also possible for the first additional polymer to be different from the first polymer. In this embodiment, an object can be coated with a blend, e.g. of polymer with the first agent bound thereto blended with the same polymer with the first additional agent bound thereto, or of a blend of different polymers, to one thereof the first agent being bound, to the other polymer the first additional agent being bound. Such a blend can be used to coat the envisaged article.

It is also possible to coat the envisaged object not with the blend, but with the separate polymer components. E.g. an inner layer can be applied from the first additional polymer with a first additional agent bound thereto, followed by application of a second outer layer comprising the first polymer with the first agent bound thereto, allowing different signals or responses to the penetration stage of the infection. For example, the inner layer can comprise an antibiotic as first additional (therapeutic) agent, whereas the outer coating may comprise a first (diagnostic) agent. Upon infection, the first agent is released from the outer layer, and as the infection progresses, the infection will arrive in the inner layer, resulting in release of the first additional agent. The same is true for a combination of first agent with first additional agent, when present on separate linker molecules.

In another attractive embodiment, a polymer molecule of the first polymer comprises, covalently attached thereto, at least one molecule of the first agent and at least one molecule of the first additional agent. Thus, both first and first additional agent are present on the same polymeric molecule, resulting in an improved concerted release of both agents.

The advantage of using a peptide linker that comprises a cleavage site is that the diagnostic agent and therapeutic agent can be located on the same polymer such that both the diagnostic agent and therapeutic agents are released concomitantly with the cleavage at the protease cleavage site.

In still another embodiment, the first additional agent is covalently attached to the at least first agent.

For example the first and first additional agents may be covalently bound to the polymer via a single linker, so that upon specific cleavage of the said cleavage site, the first and first additional agents are released by a single cleavage event. Both first and first additional agent can to this end be linked to one another, i.e. covalenty, and one of the said agents is linked to the first linker. When the first additional agent is covalently attached to the first agent, this has the benefit that both the first and first additional agents can be released simultaneously. Also, the first and first additional agents may be reacted together prior to covalently attaching to the linker thus reducing the number of reaction steps that need to be carried out on the polymer-linker construct. In the case where the first and first additional agents have two different functions, two effects will be achieved as a result of a single cleavage. The said two different functions might be a diagnostic and therapeutic function, or two different diagnostic signals, one to provide an internal reference and one to provide a means of reporting the presence of a microbial strain, species or genera.

Preferably the therapeutic agent is chosen from the group, consisting of beta-lactam antibiotics, cephalosporin antibiotics, macrolides, cyclic depsipeptides and tetracyclines, preferably being Vancomycin. Vancomycin targets the d-Ala-d-Ala motif of Gram positive bacteria and so Vancomycin would be the antibiotic of choice when coupled with a cleavage site that is recognised by gram positive bacteria.

Further examples of therapeutic agents that can be used in the invention include, but are not limited to, chlorhexidine, 2-p-sulfanilyanilinoethanol, 4,4'-sulfinyldianiline, 4-sulfanilamidosalicylic acid, acediasulfone, acetosulfone, amikacin, amoxicillin, amphotericin B, ampicillin, apalcillin, apicycline, apramycin, arbekacin, aspoxicillin, azidamfenicol, azithromycin, aztreonam, bacitracin, bambermycin(s), biapenem, brodimoprim, butirosin, capreomycin, carbenicillin, carbomycin, carumonam, cefadroxil, cefamandole, cefatrizine, cefbuperazone, cefclidin, cefdinir, cefditoren, cefepime, cefetamet, cefixime, cefinenoxime, cefminox, cefodizime, cefonicid, cefoperazone, ceforanide, cefotaxime, cefotetan, cefotiam, cefozopran, cefpimizole, cefpiramide, cefpirome, cefprozil, cefroxadine, ceftazidime, cefteram, ceftibuten, ceftriaxone, cefuzonam, cephalexin, cephaloglycin, cephalosporin C, cephradine, chloramphenicol, chlortetracycline, ciprofloxacin, clarithromycin, clinafloxacin, clindamycin, clomocycline, colistin, cyclacillin, dapsone, demeclocycline, diathymosulfone, dibekacin, dihydrostreptomycin, dirithromycin, doxycycline, enoxacin, enviomycin, epicillin, erythromycin, flomoxef, fortimicin(s), gentamicin(s), glucosulfone solasulfone, gramicidin S, gramicidin(s), grepafloxacin, guamecycline, hetacillin, imipenem, isepamicin, josamycin, kanamycin(s), leucomycin(s), lincomycin, lomefloxacin, lucensomycin, lymecycline, meclocycline, meropenem, methacycline, micronomicin, midecamycin(s), minocycline, moxalactam, mupirocin, nadifloxacin, natamycin, neomycin, netilmicin, norfloxacin, oleandomycin, oxytetracycline, p-sulfanilylbenzylamine, panipenem, paromomycin, pazufloxacin, penicillin N, pipacycline, pipemidic acid, polymyxin, primycin, quinacillin, ribostamycin, rifamide, rifampin, rifamycin SV, rifapentine, rifaximin, ristocetin, ritipenem, rokitamycin, rolitetracycline, rosaramycin, roxithromycin, salazosulfadimidine, sancycline, sisomicin, sparfloxacin, spectinomycin, spiramycin, streptomycin, succisulfone, sulfachrysoidine, sulfaloxic acid, sulfamidochrysoidine, sulfanilic acid, sulfoxone, teicoplanin, temafloxacin, temocillin, tetracycline, tetroxoprim, thiamphenicol, thiazolsulfone, thiostrepton, ticarcillin, tigemonam, tobramycin, tosufloxacin, trimethoprim, trospectomycin, trovafloxacin, tuberactinomycin, vancomycin, azaserine, candicidin(s), chlorphenesin, dermostatin(s), filipin, fungichromin, mepartricin, nystatin, oligomycin(s), ciproflaxacin, norfloxacin, ofloxacin, pefloxacin, enoxacin, rosoxacin, amifloxacin, fleroxacin, temafloaxcin, lomefloxacin, perimycin A or tubercidin, and the like.

In a particularly preferred embodiment of the present invention the first polymer, and/or, if present, the first additional polymer comprises a hydrogel. The term 'hydrogel' is used herein and is understood to have the meaning as generally understood in the prior art. A hydrogel is, therefore, a continuous solid network enveloped in a continuous liquid phase. Hydrogels possess a degree of flexibility very similar to natural tissue, due to their significant water content. Hydrogels described herein are made of branched or non-branched polymer chains that are covalently cross-linked. Hydrogels have particular advantages as the rheological properties can be tuned to suit the environment in which they are used.

In an preferred embodiment the first polymer, and/or, if present, the first additional polymer comprises a biodegradable polymer. A biodegradable polymer coating is advantageous as biodegradation of the polymer coating prevents the polymer coating residing in the patient for an extending period of time, which can cause undesired side effects, such as unwanted immune effects. By biodegradable is meant a polymer that can be broken down by the host into molecules that can be excreted by the host. Biodegradable polymers are also known in the art as absorbable polymers. By an absorbable polymer coating is meant that the polymer coating readily breaks down or degrades and is either absorbed by the body, or passed by the body. More particularly, a poly(etheylene glycol)-based polymer coating degrades to degradation products that do not elicit permanent chronic foreign body reaction, because they are absorbed by the body or passed from the body, such that no permanent trace or residual of the degradation products is retained by the body.

A wide variety of polymers can be utilized to as a first and/or first additional, polymer according to the invention as described herein, including for example both biodegradable polymers and hydrogels.

Representative examples of biodegradable hydrogels include albumin, collagen, gelatin, chitosan, hyaluronic acid, starch, cellulose and derivatives thereof (e.g., methylcellulose, hydroxypropylcellulose, hydroxypropylmethylcellulose, carboxymethylcellulose, cellulose acetate phthalate, cellulose acetate succinate, hydroxypropylmethylcellulose phthalate), alginates, casein, dextrans, polysaccharides, fibrinogen, poly(L-lactide), poly(D,L lactide), poly(L-lactide-co-glycolide), poly(D,L-lactide-co-glycolide), poly(glycolide), poly(trimethylene carbonate), poly(hydroxyvalerate), poly(hydroxybutyrate), poly(caprolactone), poly(alkylcarbonate) and poly(orthoesters), polyesters, poly(hydroxyvaleric acid), polydioxanone, poly(malic acid), poly(tartronic acid), polyanhydrides, polyphosphazenes, poly(amino acids), copolymers of such polymers and blends of such polymers (see generally, Ilium, L., Davids, S. S. (eds.) "Polymers in Controlled Drug Delivery" 1987, Wright, Bristol,; Arshady, 1991, J. Controlled Release 17:1-22,; Pitt, Int. J. Phar. 59:173-196, 1990; Holland et al., 1986, J. Controlled Release 4:155-180).

Poly(ethylene glycol) with multifunctional sites for conjugating the cleavage site containing linker to the hydrophilic polymer coating as well as cross-linking the hydrophilic polymer coating also find use in the present invention. Multifunctionally activated synthetic polymers can be prepared using various techniques known in the art which provide functional groups at various locations along the polymer.

Poly(ethylene glycol) diacrylate (PEGDA) is a synthetic, hydrophilic starting material which forms hydrogels in the presence of photoinitiator and UV light. PEGDA hydrogels are easily customizable since extra cellular matrix proteins and/or growth factors can be incorporated into a hydrogel and its stiffness can be modulated from 10-100 kPa PEGDA is widely recognized as a biocompatible, non-immunogenic, and capable of chemical manipulation to incorporate attachment protease cleavage sites and therapeutic or diagnostic agents.

Poly(ethylene glycol) diacrylate (PEGDA) hydrogels have been widely accepted in many biomedical applications (Peppas, et al., 1999, J. Controlled Release 62:81-87). Hydrogels prepared using, e.g., PEG(574)DA, PEG(4000)DA and PEG(8000)DA, have been demonstrated herein to have adequate physical characteristics for implantation and manipulation prior to placement. PEGDA hydrogels are hydrophilic, biocompatible, nontoxic, and exhibit variable mesh size depending upon PEG macromer length. As exemplified herein, hydrogels containing the first agent and/or first additional attached via a first cleavage site peptide provide a level of control for the release the first agent And/or first additional agent.

As disclosed herein, large PEG chain lengths allow proteins to diffuse throughout the matrix while smaller PEG chain lengths can be used to control the accessibility of the matrix to specific proteins (Elbert, et al.,2001, J. Controlled Release 76:11-25). In this regard, certain embodiments embrace a hydrophilic matrix with a mesh size which allows bacterial proteases to diffuse throughout the polymer coating.

In addition to functional groups, the polymers of the polymer coating can further contain a means for controlled biodegradation to facilitate removal of the matrix polymer from the subject being treated. For example, PEGDA hydrogels can be made to biodegrade at a faster rate by modification (Sawhney, et al.,1994, J. Biomed. Mater. Res. 28:831-838). PEGDA hydrogels can be made biodegradable by incorporating a biodegradable cross linker or by utilizing biodegradable copolymers (Sawhney, et al.,1993, Macromolecules 26:581-587; Park, et al. Biodegradable Hydrogels for Drug Delivery.,1993, Lancaster, Pa.: Technomic Pub. ix, 252; Watanabe, et al., 2002, Biomaterials 23:4041-4048; Yamini, et al., 1997, J. Macromol. Sci. A34:2461-2470). For example, telechelic (that is a polymer capable of entering into further polymerization or other reactions through its reactive end-groups) biodegradable block copolymers, specifically degraded by either plasmin or crude collagenases, have been used in cross-linked hydrogels (West, et al., 1999, Macromolecules, 32:241-244). The extent and rate or degradation is controlled by the specific degradation mechanism used thereby limiting accumulation of the hydrophilic polymer coating at the site of implantation (US2007/0155906).

Immobilization of an agent to the polymer coating (for example to a hydrogel) via thiol conjugation is a favourable approach given the ease of cysteine incorporation within a peptide linker, making the thiolene click reaction particularly relevant for hydrogel surface modification.

Anseth and co-workers, (2009, Nat Mater., 659-664) developed a sequential click protocol relevant to both hydrogel synthesis and postgelation modification. Click cross-linked PEG hydrogels were first formed via CuAAC, as an extension of the method taken by Malkoch et al (2006, Chem. Commun, 2774-2776). To enable photopatterning of their PEG hydrogels, multifunctional photoreactive polypeptide sequences were included within the network structure by incorporating the non-natural amino acid, Fmoc-Lys(alloc)-OH. The allyloxycarbonyl (alloc) protecting group contains a vinyl functional group capable of reacting with any thiol-containing compound, such as cysteine. Upon exposure to UV light, thiyl radicals are generated via the photocleavage of a hydrogen-abstracting initiator, thereby using light to achieve spatial and temporal control of thiolene functionalization within the network. To illustrate this technique, a fluorescently labeled cysteine containing peptide was patterned within PEG hydrogels via transparency-based photolithographic patterning techniques. This thiolene photopatterning method was later employed to immobilize peptides and proteins within PEG hydrogels cross-linked via spatially activated click coupling (SPAAC). Spatial and temporal control was validated by selectively exposing certain locations within the hydrogel matrix to light, and by controlling light intensity and exposure time.

. Representative examples of non-degradable polymers include poly(ethylene-co-vinyl acetate) ("EVA") copolymers, silicone rubber, acrylic polymers (e.g., polyacrylic acid, polymethylacrylic acid, poly(hydroxyethylmethacrylate), polymethylmethacrylate, polyalkylcyanoacrylate), polyethylene, polyproplene, polyamides (e.g., nylon 6,6), polyurethane (e.g., poly(ester urethanes), poly(ether urethanes), poly(ester-urea), poly(carbonate urethanes)), polyethers (e.g., poly(ethylene oxide), poly(propylene oxide), Pluronics and poly(tetramethylene glycol)) and vinyl polymers [e.g., polyvinylpyrrolidone, poly(vinyl alcohol), poly(vinyl acetate phthalate)]. Polymers may also be developed which are either anionic (e.g., alginate, carrageenin, carboxymethyl cellulose and poly(acrylic acid), or cationic (e.g., chitosan, poly-L-lysine, polyethylenimine, and poly (allyl amine)) (see generally, Dunn et al., J. Applied Polymer Sci. 50:353-365, 1993; Cascone et al., J. Materials Sci.: Materials in Medicine 5:770-774, 1994; Shiraishi et al., Biol. Pharm. Bull. 16(11):1164-1168, 1993; Thacharodi and Rao, Int'l J. Pharm. 120:115-118, 1995; Miyazaki et al., Int'l J. Pharm. 118:257-263, 1995).

Particularly preferred polymeric carriers include poly(ethylene-co-vinyl acetate), polyurethane, acid, poly(caprolactone), poly(valerolactone), polyanhydrides, copolymers of poly(caprolactone) or poly(lactic acid) with a polyethylene glycol (e.g., MePEG), and blends thereof.

In a particularly preferred embodiment of the present invention the first polymer, and/or, if present, the first additional polymer a comprises a polyethylene gylcol (PEG), preferably polyethylene glycol vinyl-sulfone more preferably polyethylene glycol diacrylate (PEGDA). PEG based polymer coatings have particularly favourable biodegradable properties and are facile to synthesize using techniques known in the art and therefore form very good material choices for the present invention.

PEG polymer hydrogels can be synthesized by standards methods known to the person skilled in the art, for example a thiol-yne reaction, ensuring a stoichiometric excess of alkynes. Postgelation, a solution of photoinitiator (Irgacure 2959), copper sulfate, and an azide-labeled diagnostic or theraepeutic agent can be swollen into the gel. Upon irradiation with a photomask, Cu(I) is generated within the irradiated areas, catalyzing the pCuAAC reaction between the azide-labeled diagnostic or therapeutic agent and the pendant alkynes in the polymer network, ultimately producing a spatially defined fluorescent pattern within the hydrogel.

In a very attractive embodiment, the material comprises a polyethylene glycol acrylate or a polyethylene glycol vinyl-sulfone. Particularly, absorbable poly(ethylene glycol) (PEG)-based hydrogels comprising the reaction product of a multi-arm-PEG-vinylsulfone (-VS) having from about 3 arms to about 8 arms and a multi-arm-PEG-R-sulfhydryl (-SH) (-SH is also known as thiol) having from about 3 arms to about 8 arms and where R is an ester linkage including, but not limited to carboxylate ester (also known as ester), lactate ester (also known as lactic ester), and isobutyrate ester (also known as isobutyric ester). This absorbable PEG-based hydrogel is useful in the field of drug delivery, where the in situ formed hydrogel can entrap a protein and release the agent over time as the hydrogel degrades, which is described in further detail below. By utilizing the methods described herein to incorporate a cleavage site peptide in the polymer coating, it is possible to prepare a polymer coating suitable for coating an object wherein the at least first agent is released in the presence of a microbe belonging to a group consisting of Gram positive bacteria.

The multi-armed PEG-VS and the multi-armed PEG-SH may be tailored to increase or decrease the crosslink density, the molecular weight, and the rate of degradation of the absorbable PEG-based hydrogel. The crosslink density may be varied by increasing or decreasing the number of PEG arms. The molecular weight of the PEG arms may also be varied. The molecular weight of the multiarm-PEG must be such that when the hydrogel degrades into degradation products the degradation products may be cleared by the kidney. Additionally, the type of ester linkage may be chosen to vary how long it takes for the hydrogel to breakdown.

The multi-arm-PEG-VS may have from about 3 arms to about 8-arms. In one embodiment, the multi-arm-PEG-VS has 4-arms. In another embodiment, the multi-arm-PEG-VS has 8 arms. The multi-arm-PEG-VS may have molecular weight of about 10 kDa to about 40 kDa. In one embodiment, the multi-arm-PEG-VS may have a molecular weight of about 10 kDa.

The multi-arm-PEG-R-SH may have from about 3 arms to about 8-arms, where R is an ester linkage including, but not limited to carboxylate ester, lactate ester, and isobutyrate ester. In one embodiment, the multi-arm-PEG-R-SH has 4-arms. The multi-arm-PEG-R-SH may have molecular weight of about 2,000 Da to about 40 kDa. In one embodiment, the multi-arm-PEG-R-SH may have molecular weight of about 10 kDa.

The PEG-based hydrogel is formed by the Michael addition reaction of multi-arm-PEG-VS with the multi-arm-PEG-R-SH. The multi-arm-PEG-VS and multi-arm-PEG-R-SH are each dissolved in separate aqueous solutions in a concentration of from about 5% (w/v) to about 40% (w/v). In one embodiment, multi-arm-PEG-VS and multi-arm-PEG-R-SH are each dissolved in separate aqueous solutions in a concentration of about 20% (w/v). The aqueous solution is defined as water or buffered water, including, but not limited to phosphate buffered saline, citrate buffer, and boric acid based buffer. In the case of a buffered water solution the pH is in the range of from about 5.5 to about 11.0. In one embodiment, the pH is in the range of from about 7.4 to about 8.5.

The separate multi-arm-PEG-VS and multi-arm-PEG-R-SH solutions are then mixed together and react to form the absorbable PEG-based hydrogel. The amount of multi-arm-PEG-VS solution mixed with multi-arm-PEG-R-SH solution is calculated such that the mole ratio of multi-arm-PEG-VS to multi-arm-PEG-R-SH is from about 1:1 to about 1:2. In one embodiment, the mole ratio of multi-arm-PEG-VS to multi-arm-PEG-R-SH is about 1:1.The polyethylene glycol (PEG) based hydrogels described herein are also suitable for formation of at least a portion of a medical device. The hydrogels may be tailored to possess adhesive properties, making them useful in the field of implantable medical devices.

A further reactive PEG which can be used to prepare the object according to the invention is a multifunctional activated synthetic polymer is monomethoxy-polyethylene glycol (mPEG), which can be activated by the addition of a compound such as cyanuric chloride, then coupled to, e.g., a linker comprising a cleavage site. Another form of activated PEG is PEG-succinate-N-hydroxysuccinimide ester (SS-PEG) (see Abuchowski, et al.,1984, Cancer Biochem. Biophys. 7:175). Activated forms of PEG such as SS-PEG react with linkers (specifically peptide linkers) under relatively mild conditions and produce conjugates without destroying the specific biological activity and specificity of the peptide or the at least first agent and /or the at least second agent attached to the PEG polymer coating.

The object is preferably chosen from the group, consisting of, medical implants.. The term 'implant' as used herein is understood to mean an object suitable for implantation in a human or animal. Said object may be solid, wherein solid is understood to mean any object that displays a surface to be covered, for example the object may comprise open sides, porous sides or be non-porous in nature.

The invention is particularly applicable in the case of medical implants, i.e. objects intended to be implanted in a patient's body during invasive surgery. A patient can be a human or any animal subject suitably for receiving an implant. Examples of medical implants include, but are not limited to, cardiovascular devices (e.g., implantable venous catheters, venous ports, tunneled venous catheters, chronic infusion lines or ports, including hepatic artery infusion catheters, pacemakers and implantable defibrillators, neurologic/neurosurgical devices (e.g., ventricular peritoneal shunts, ventricular atrial shunts, nerve stimulator devices, dural patches and implants to prevent epidural fibrosis post-laminectomy, devices for continuous subarachnoid infusions); gastrointestinal devices (e.g., chronic indwelling catheters, feeding tubes, portosystemic shunts, shunts for ascites, peritoneal implants for drug delivery, peritoneal dialysis catheters, and suspensions or solid implants to prevent surgical adhesions); genitourinary devices (e.g., uterine implants, including intrauterine devices (IUDs) and devices to prevent endometrial hyperplasia, fallopian tubal implants, including reversible sterilization devices, fallopian tubal stents, artificial sphincters and periurethral implants for incontinence, ureteric stents, chronic indwelling catheters, bladder augmentations, or wraps or splints for vasovasostomy, central venous catheters, urinary catheters; prosthetic heart valves vascular grafts, opthalmologic implants (e.g., multino implants and other implants for neovascular glaucoma, drug eluting contact lenses for pterygiums, splints for failed dacrocystalrhinostomy, drug eluting contact lenses for corneal neovascularity, implants for diabetic retinopathy, drug eluting contact lenses for high risk corneal transplants); otolaryngology devices (e.g., ossicular implants, Eustachian tube splints or stents for glue ear or chronic otitis as an alternative to transtempanic drains); plastic surgery implants (e.g., breast implants or chin implants), catheter cuffs and orthopedic implants (e.g., cemented orthopedic prostheses). In an attractive embodiment, the implant is a k-wire.

Any object of interest can be coated. Objects, in particular surfaces thereof, that are intended to remain sterile can be coated with the material of the invention. For example, if an object, coated with a material according to the invention where the material incorporates a photon-emitting agent emitting light upon release from the material, is contaminated with a specific microbe, the material of the invention will release the photon-emitting agent as a result of which light will be emitted from the material. Contamination of the object is observable using suitable detectors.

A further advantage of the method according to the invention is in the monitoring of a patient post surgery to implant a medical device. For example, after phasic intraocular lens implantation, an infection can easily be detected by monitoring for fluorescence emitted from the lens.

The use of photon emitting - agents offers a flexible and direct imaging methodology. The photon emitting - agents can be visualized by excitation with an appropriate light source and capture of the emitted photons with a CCD camera or other optical detector. In fluorescent imaging, there are generally three parameters used to characterize the interaction of photons with tissues: light absorption, light scattering, and fluorescent emission. One of the most important considerations in optical imaging is maximizing the depth of tissue penetration. Absorption and scattering of light are largely a function of the wavelength of the excitation source. Light is absorbed by endogenous chromophores found in living tissue, including hemoglobin, melanin, and lipid. In general, light absorption and scattering decrease with increasing wavelength. In particular, the absorption coefficient of tissue is considerably lower in the near infrared (NIR) region (700-1,000 nm) and so light in the NIR can penetrate more deeply, to depths of several centimeters. Agents with emissions in the NIR are not hindered by interfering auto fluorescence, so they tend to yield the highest signal-to-background. Such an agent is, for example, but not limited to, a near-infrared fluorescent agent such as IRDYE800.

In yet another embodiment of the invention, the invention relates to a method of producing a coated object according to the invention, comprising the steps of
a) dip coating a medical implant in a solution of a polymer comprising
one or more polymers, said polymers comprising a first cleavage site and a first agent releasable from said coating upon cleavage of said first cleavage site,
wherein the first agent is covalently bound to a first polymer comprising polyethylene glycol via a first linker, the first linker comprising the first cleavage site being a peptide,
wherein the first cleavage site is cleaved by a sortase enzyme specifically provided by a microbe belonging to a first group consisting of a Gram positive bacteria, and not cleaved by any compound provided by any microbe not belonging to said first group,
wherein cleavage of the said first cleavage site results in release of the said first agent from the coating, the release of said first agent being indicative for the presence of a Gram positive bacteria,
wherein the first agent is a diagnostic agent being a fluorescent agent, b) drying the dipped object.

An object according to the present invention may be coated with said polymer coating using techniques such as dip - coating, spray - coating, spin - coating, or solvent casting. Coating techniques involving the chemical grafting of molecules onto the biomaterial surface are also available. Nano-thin coatings based on self - assembled monolayers (SAMs), surface - tethered polymers (polymer brushes), or multilayer coatings based on layer - by - layer assembly offer precise control on the location and orientation of chemical groups and biomolecules on the surface of the coating are commonly known in the art to apply various coatings to orthopedic components and other medical devices for a variety of reasons,. See, Handbook of Materials for Medical Devices, Davis, J. R. (Ed.), Chapter 9, "Coatings" 2003.

The polymer coating may be partial or complete with respect to the surface area of the means for providing at least a primary function.

Furthermore the technologies developed by InnoCore (www.innocore.nl) are suitable for the polymer coatings according to the present invention. For example, compositions comprising DL-lactide, glycolide, ε-caprolactone and polyethyleneglycol are biologically safe monomers that have been approved for human in vivo applications and are already used in numerous marketed biomedical implants and pharmaceutical sustained release formulations. Such drug-eluting coatings can be applied on a broad variety of biomedical implant devices for the controlled and local delivery of various types of drug molecules. Typical applications include coronary stents and other vascular devices, orthopedic prostheses and urinary implants.

Tests on the coating may be carried out according to Standard Test Method for in vitro Degradation Testing of Hydrolytically Degradable Polymer Resins and Fabricated Forms for Surgical Implants ASTMF1635-11;

The invention also relates to a polymer coating, the coating comprising
a) one or more polymers wherein said polymers comprise a first cleavage site and
b) a first agent releasable from said coating upon cleavage of said first cleavage site
characterized in that the first agent is covalently bound to a first polymer comprising polyethylene glycol via a first linker, the first linker comprising the first cleavage site being a peptide,
wherein the first cleavage site is cleaved by a sortase enzyme specifically provided by a microbe belonging to a first group consisting of a Gram positive bacteria, and not cleaved by any compound provided by any microbe not belonging to said first group, wherein cleavage of the said first cleavage site results in release of the said first agent from the coating, the release of said first agent being indicative for the presence of a microbe belonging to the said first group, wherein the first agent is a diagnostic agent being a fluorescent agent.

As discussed above, the first agent and / or first additional agent, can be covalently linked to the polymer coating of the invention In both cases, release is effected by the specific cleavage of the polymer coating induced by the microbe. The first agent and / or first additional agent can e.g. be embedded in a polymer coating, wherein said polymer coating can be said to have a web-type structure. The said web-type structure can be disintegrated by the specific cleavage site as described earlier based on the compound specific for a microbe, for example an enzyme, for example a sortase.

The invention also relates to the use of an object according to the invention monitoring and/or treating a microbial infection, in particular a biofilm associated infection. The presence and/or development of an infection, in particular a biofilm associated infection, can be monitored. A biofilm of microbes can be formed on the surface of object according to the invention. Release of the agent from the polymeric coat of the object will therefor be indicative for the presence of such a biofilm, and can e.g. be determined by irradiation of the object, such as a working surface, or an implant where irradiation can take place on the outer surface of a patient carrying the implant and by detection of the luminescence *in situ.*

The invention also relates to the use of a polymer coating according to the invention, suitable for coating an object, in particular an implantable device for the monitoring for monitoring the presence and/or development of an infection, in particular a biofilm associated infection, and/or treatment thereof. The polymer coating according to the invention is ideally suited to monitoring and or treating a biofilm associated invention as the said polymer releases a particular agent that is indicative for the presence of a particular microbe or group of microbes. Application of the coating renders the object therefore very suitable for detection of a particular microbial infection thereon.

The invention will be illustrated by the following non limiting examples and drawings, showing:
in figure 1, a schematic diagram of a coating according to the invention wherein an agent is covalently coupled to the polymeric matrix via linker molecules.
in figure 2 a schematic diagram of a coating not according to the invention having a polymeric web with the agent embedded therein.
in figure 3, a schematic diagram of an object coated with a polymer coating according to the invention.
in figure 4, a schematic diagram of the object of figure 3 in the presence of a particular microbe that produces a compound specific for the cleavage site in the polymer coating.
in figure 5, a schematic diagram of the object of figure 4 upon irradiation.
in figure 6, a photograph of an orthopaedic plate coated with a polymer coating according to the invention, implanted in a human ankle, before closing of the wound.
in figure 7 a fluorescent image of the implant of figure 6 after closure upon irradiation by an external light source.
in figure 8 a photograph of an indwelling catheter coated with a polymer coating according to the invention, subcutaneously implanted in a human ankle.
in figure 9 a fluorescent image of the implant of figure 8 upon irradiation external light source.
in figure 10 a photograph of an orthopaedic plate and a catheter both coated and implanted in a human ankle (control implants).
in figures 11 a fluorescent image of the implants of figure 10 upon irradiation external light source.

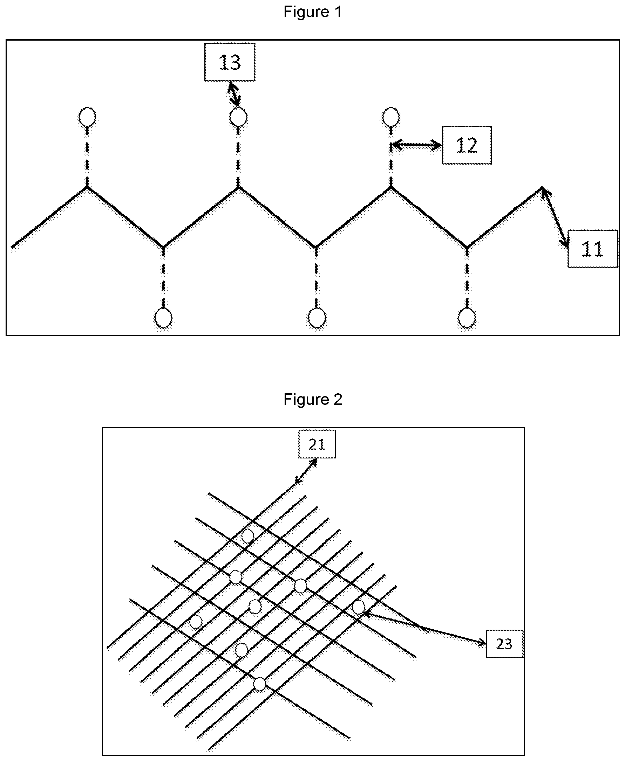

In figure 1, a polymer, for example poly(ethylene glycol) is depicted by a solid line 11. Linker molecules, for example oligopeptides, depicted with dotted lines are covalently linked to the polymer. The linker molecules comprise a cleavage site, cleavable by one or more compounds, provided by one or more microbes. Said linkers 12 can e.g. be amino acid motifs comprising a specific portion of molecular structure, recognizable by e.g. a protease produced and excreted by a bacterium of group of bacteria. The linker is covalently bound to a first agent 13, e.g. a diagnostic agent such as a photon emitting agent, or a therapeutic agent, such as an antibiotic.

In figure 2, a polymer coating 21 is shown, which has a web structure. Within the web, an agent, such as a diagnostic or therapeutic agent 23 is entrapped. The agent is bound non-covalently in the polymer coating. The web structure comprises specific protease cleavage sites, cleavable by specific microbes. Cleavage will result in release of the agent 23 from the polymer coating 21.

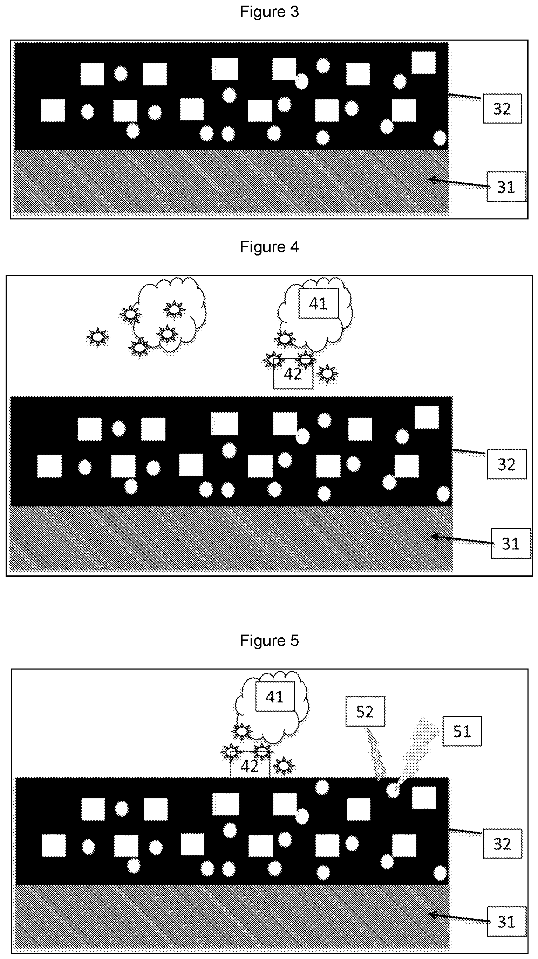

In figure 3 a surface portion of an object 31, such as an implantable object or a working surface, is coated with a layer of polymer coating 32 of the invention comprising a first agent 33 (circles) and a second agent 34 (squares). Both first an second agents can be diagnostic molecules, preferably different from one another, i.e. giving a different signal upon release, or, accordingly, can be both a therapeutic agent. It is also possible for the first agent to be a diagnostic agent, and for the second agent to be a therapeutic agent, or vice versa. Both first an second agents can be embedded in the polymer as shown in figure 2, or be covalently linked to the polymer via linker molecules. In case the first agent and the second agent are both diagnostic agents differing in signalling upon release thereof from the coating, it is preferred to have these agents covalently linked to the polymer via different linker molecules. The first agent is in that case linked to the polymer via a first linker, and the second agent by a second linker. The first linker has a first cleavage site, different from the second cleavage site, present on the second linker. The first and second cleavage sites of the first and second linker, respectively, are preferably different from one another, so that different microbes, i.e. microbes that belong to two different groups as defined herein, each are capable of cleaving either the first or second cleavage site. Release of the first agent is indicative for the presence of a microbe belonging to the first group, and the release of the second agent is indicative for the presence of a microbe of the second group. By using different diagnostic agents, such as chemoilluminescent agents having different colour upon excitation with light of a defined wave length, for the first and second agents, a difference in colour is discriminative for the presence of a microbe belonging to the first or second group.

In figure 4, an object as in figure 3 is infected by microbes 41, such as pathogenic bacteria. In this case, the circles represent the first agent (circles) and can be a diagnostic, and the squares represent a first additional agent or a second agent, preferably being a therapeutic agent such a an antibiotic against the said microbe. Both first and second agent are coupled to the polymer of the coating via the same linker molecules, i.e. having the same cleavage site. In can be chosen to link both the first and the first additional agent to the very same first single linker molecule, so that both agents are released upon a single cleavage by a compound 42 (stars) of the microbe 41 (clouds). It can also be arranged such, that each linker molecule is only coupled to either the first agent or the first additional agent, and that a mixture of these differentially coupled linkers is coupled to the polymer of the coating. In both cases, both the first and first additional agents are released by cleavage by the same compound 42. Cleavage will result in release of the fist (e.g. diagnostic) agent 33 and a first additional (e.g. therapeutic) agent 34. The infectious microbe can easily be detected upon infection by release of the diagnostic agent, whereas the infection is simultaneously be counteracted by the release of the therapeutic agent.

It can also be chosen to coat an object first with a first polymer coating comprising the first agent, followed by a second coating on the first coating, the second coating comprising the second agent or the first additional agent (or a combination thereof). In this arrangement, the infection by a microbe first arrives at the second coating, releasing the second agent, e.g. a diagnostic agent. In case the infection continues, it will arrive at the first coating, resulting in the first agent, such as a therapeutic like an antibiotic, to be released.

In figure 5, the infected object of figure 4 is irradiated with external light 51. Released first (diagnostic) agent (circles) is activated by the external light 51 and excites light of a different wavelength 52 as that of the external light 51. The said emitted light 52 can e.g. be near infrared light (NIRF). The irradiation makes the infection visible, while the released first additional agent (antibiotic) already acts to eliminate the pathogenic bacteria

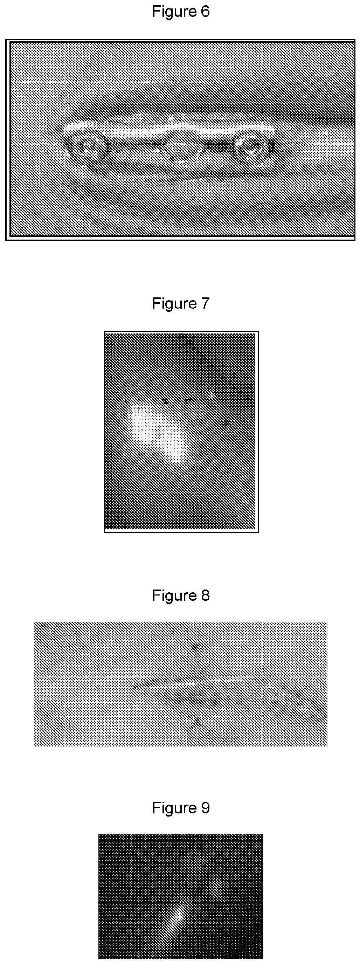

In Figure 6 a metallic orthopaedic device, coated with a coating of the invention is shown fixed onto bone in a human ankle, just before closing of the wound.

Figure 9 shows an image taken on an IVIS (in vivo imaging station) camera at the site of the implant of figure 6 after infection with microbes and irradiation with an external light source. For further details, see Example 8. The emitted light indicates the presence of a microbial infection.

Figure 8 shows an indwelling catheter, subcutaneously arranged at a human ankle.

Figure 9 shows an fluorescent image taken on an IVIS camera at the site of the implant of figure 8 after infection and irradiation with an external light source according to Example 8. The emitted light indicates the presence of a microbial infection.

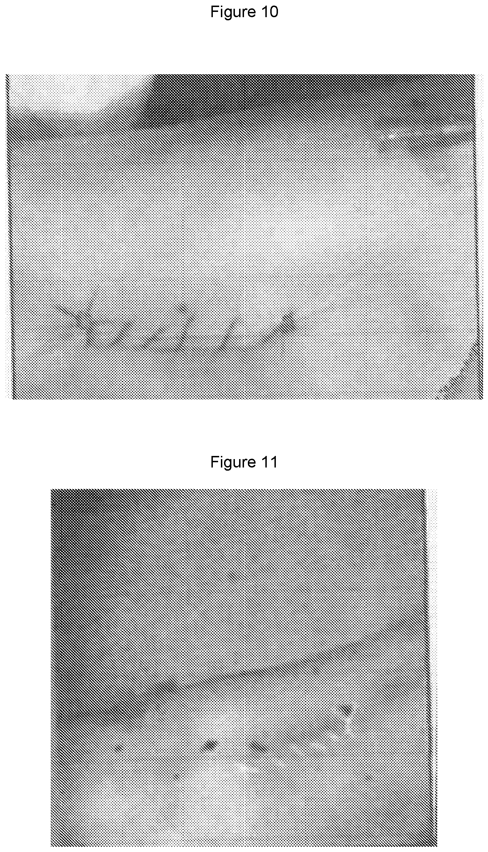

Figure 10 shows a photograph of an orthopaedic plate and a catheter implanted in a human ankle after closure of the wound.

Figure 9 shows an fluorescent image taken on an IVIS camera at the site of the control implants of figure 8 after irradiation. The lack of an emitted signal is indicative for the lack of an infectious agent.

### EXAMPLES

### Example 1

### Preparation of polymeric coatings

### a) Synthesis of polymer.

The polymer PEGDA was synthesized using PEG(4000) or PEG(8000) and acryloyl chloride (Hern and Hubbell ,1998, J. Biomed. Mat. Res. 39(2):266-276).

### b) Synthesis of linkers

Two peptide linkers were made using FMOC solid phase peptide chemistry (Fmoc Solid Phase Peptide Synthesis: A Practical Approach (Practical Approach Series), Chan and White, Oxford University Press, 1999)
i) CGGGLPETGGSGGK (SEQ NO:15) contains a first cleavage site, LPTEG, which is cleaved by Sortase A from *S. aureus.*
ii) CGGGAAFGGSGGK (SEQ NO:15) contains a second cleavage site, AAF, which is cleavable by serralysin from *P. aeruginosa.*

The peptides were cleaved from the resin using standard conditions (trifluoroacetic acid, 95%). Peptides were purified (> 97% pure) by reverse phase high-performance liquid chromatography. Peptide structures can be confirmed by liquid chromatography-mass spectrometry.

### c) Synthesis of a first agent and a first additional agent, IRDye800 covalently bound to vancomycin.

Vancomycin labeled with IRDye®800CW was provided by LI-COR Biosciences (Lincoln NE, USA). Vancomycin-IRDye®800CW (vanco-800CW) was synthesized by an adapted literature procedure. Vancomycin hydrochloride hydrate (3.0 mg, 2.0 µmol) was added to a solution of IRDye®800CW-NHS ester (1.0 mg, 0.86 µmol) and N,Ndiisopropylethylamine (2.0 µL, 11 µmol) in dimethylsulfoxide (200 µL). After overnight reaction at ambient temperature, the resulting bioconjugate was purified by reverse phase HPLC and lyophilized to afford a green flocculent solid (1.0 mg, 48%). Within the limits of HPLC detection, the final product did not contain unconjugated dye (detected at 780nm) or unlabeled vancomycin (detected at 280nm). UV/Vis (methanol) λmax = 778nm; Low Resolution Mass Spectrometry (ES/ammonium formate), m/z calculated for 2430.7 [M - H]-, found 810.6 [M - 3H]³⁻.

### d) Conjugate synthesis: first linker with a first cleavage site - first agent and first additional agent

i) A 1 g batch of the peptide linker as prepared above (step (b)(i)) was labeled on the the lysine (K) side chains with a first agent, IR dye 800CW NHS ester, (LI-COR Biosciences (Lincoln NE, USA)) using literature procedures (Tiyanont, K, et al. Proc. Natl. Acad. Sci. USA 2006, 103,11033-11038).
ii) A second 1 g batch of the peptide linker as prepared above (step (b)(i)) was labelled on the serine (S) side chains with a first additional agent, Vancomycin, (used as supplied by Sigma Aldrich) using standard coupling methods (Fmoc Solid Phase Peptide Synthesis: A Practical Approach (Practical Approach Series), Chan and White, Oxford University Press, 1999).
iii) A third 1 g batch of the peptide linker as prepared above (step (b)(i)) was labeled with both a first agent, IRDye800 and a first additional agent, vancomycin.
iv) A fourth 1 g batch of the peptide linker prepared above (step (b)(i)) was labeled with the product of step (c), the IRDye800-vancomycin covalent conjugate.

### e) Synthesis of polymer - linker conjugates

The three peptide linkers as prepared above in steps (d)(i), (ii) and (iii) were each covalently bound to the PEGDA polymer synthesized in step (a) by adding the peptide linker (2 mM) to the PEGDA (8 mM-100 mM) in phosphate-buffered saline (pH 7.4) and stirring for 4 hours to overnight. Michael addition of the acrylate group to the sulfhydryl group on cysteine (Heggli, et al. (2003) Bioconjugate Chem. 14:967-973) was confirmed using Ellman's reagent (Pierce, Milwaukee, Wis.), thereby quantifying reduction in free sulfhydryl groups (Ellman (1959) Arch. Biochem. Biophys. 82:70-77). The solution was then polymerized using ammonium persulfate (APS, 20 mM) and N,N,N,N-tetramethylethylenediamine (TEMED, 51.6 mM) at 37°C. The products of the these coupling reactions are summarized in Table 1.

| Conjugate | Polymer | First Linker (first cleavage site in bold) | First agent (covalently bound to K) | First additional agent (covalently bound to S) |
|---|---|---|---|---|
| 1 e (i) | PEGDA₄₀₀₀ | CGGG**LPET**GGSGGK | IRDye800 | - |
| 1 e (ii) | PEGDA₄₀₀₀ | CGGG**LPET**GGSGGK | - | Vancomycin |
| 1 e (iii) | PEGDA₄₀₀₀ | CGGG**LPET**GGSGGK | IRDye800 | Vancomycin |
| 1 e (iv) | PEGDA₄₀₀₀ | CGGG**LPET**GGSGGK | IRDye800-Vancomycin | |
| 1 e (vi) | PEGDA₈₀₀₀ | CGGG**LPET**GGSGGK | IRDye800 | - |
| 1 e (vii) | PEGDA₈₀₀₀ | CGGG**LPET**GGSGGK | - | Vancomycin |
| 1 e (viii) | PEGDA₈₀₀₀ | CGGG**LPET**GGSGGK | IRDye800 | Vancomycin |
| 1 e (ix) | PEGDA₈₀₀₀ | CGGG**LPET**GGSGGK | IRDye800-Vancomycin | |

### f) Conjugate synthesis of a linker with a second cleavage site- second agent- comparative example

A 1 g batch of the peptide linker as prepared above (step (b)(ii)) was labeled on the serine (S) side chains with a second agent, indocyanin green (LI-COR Biosciences (Lincoln NE, USA)) using literature procedures (Villaraza, A. , et al. Bioconjugate Chem., 2010, 21 (12), pp 2305-2312) as shown in Table 2.

**Table 2**

| Conjugate | Second Polymer | Second Linker (second cleavage site in bold) | Second agent |
|---|---|---|---|
| 1 f (i) | PEGDA₄₀₀₀ | CGGG**AAF**GGSGGK | indocyanin green |
| 1 f (ii) | PEGDA₈₀₀₀ | CGGG**AAF**GGSGGK | indocyanin green |

### g) Preparation of polymer coatings

Using the products from step (e) a number of different polymer coatings were prepared by simply mixing different proportions of conjugates 1 e (i) - (ix) and 1 f with each other.

A polymer coating (1 g (i)) was prepared that containing a polymer comprising a first cleavage site and a first agent (1 e (i)), and polymer comprising a first cleavage site and a first additional agent (1 e (ii)). In this way the first agent (IRDye800) and the first additional agent (vancomycin) can therefore be present in equal or different proportions, for example the said coating was prepared with 50 % IRDye and 50 % vancomycin.

Furthermore, any of conjugates 1 e (i) - (iv) can be mixed in with any of the conjugates 1 e (vi)-(ix). In this way a polymer coating (1 g (ii)) comprising 50% of a first polymer (PEGDA4000) comprising a first agent (IRDye800) (conjugate 1 e (i)) was mixed with 50 % of a second polymer (PEGDA8000) comprising a first additional agent (vancomycin) (conjugate 1 e (vii)). The resultant polymer coating therefore had the advantage of containing a two types of PEGDA polymer so that the rheological properties of the coating that was made from the two polymers could be optimized.

By way of a further example a polymer coating (1 g (iii) was prepared that contained 50 % of conjugate 1 e (i) and 50 % of 1 f. This polymer coating contained a first cleavage site, LPETG (SEQ NO:5), cleavable by sortase A from *S*. *aureus* so that IRDye800 can be released in the presence of S. aureus and a second cleavage site AAF, cleavable by serralysin from *P. aeruginosa,* so that indocyanin green can be released in the presence of *P. aeruginosa.* In this way the coating 1 f (v) can detect the presence of two specific microbes, namely *S. aureus* and *P. aeruginosa.*

Conjugates 1 e (iv) and 1 e (ix) enable the preparation of polymer coatings wherein the first agent and first additional agent are covalently bound to each other. In such a coating, the first and first additional agents are therefore released from the polymer coating at precisely the same location when a first cleavage site is cleaved by a compound specific for a microbe.

Different coatings can also be coated on an object in a subsequent manner, resulting in different coating layers as explained above.

### Example 2

### Coating comprising an agent embedded in a polymer web

### a) Synthesis of linker

A different linker to that used in Example 1 is necessary in order to synthesize a polymer coating where the linker is incorporated within the polymer matrix itself.
The linker (N₃)GGGLPETGGSGGK(N₃) was synthesized using Fmoc solid phase peptide synthesis as described above, using the modified amino acid building blocks (N₃) and K(N₃) as described in Van Dijk et al., Biomacromolecules, 2010, 11, 1608-1614.

### b) Preparation of polymer-linker-polymer matrix

The polymer matrix wherein linkers comprising cleavage sites are incorporated within the hydrogel matrix itself was synthesized by the method of van Dijk, namely the Cu(I)-catalyzed 1,3-dipolar cycloaddition reaction between a cleavage site containing bis-azido peptide linker prepared in above in Example 2 step (a) and star-shaped alkyne-derivatized PEG moieties (Van Dijk et al, *supra*). The peptide linker contains the LPETG (SEQ NO:5) cleavage site that is cleaved by Sortase A from *S*. *aureus.*

### c) Incorporation of first agent and first additional agent in a polymer matrix

i) The first agent (IRDye800), and the first additional agent (vancomycin) or the first agent-first additional agent conjugate as prepared in Example 1 step (c), were non-covalently incorporated into the hydrogel matrix by soaking in a solution of the polymer matrix. The products of these reactions are summarized in table 3. The products described in Table 3 can be used to prepare polymer coatings were the first and or first additional agents are therefore released from the polymer coating when sortase A, specific for the LPETG (SEQ NO:5) motif, cleaves the cleavage site present in the polymer matrix itself.

**Table 3**

| Polymer matrix | First agent | First additional agent |
|---|---|---|
| 2 c (i) | IRDye800 | - |
| 2 c (ii) | IRDye800 | vancomycin |
| 2 c (iii) | IRDye800-vancomycin | - |

### Example 3

### Preparation of a coated orthopedic plate (figure 3).

Orthopaedic plates, screws and catheters were coated with the polymer coating with compositions according to step (g). For example a polymer coating 1 g (i) was coated onto orthopaedic plates, screws and catheters in a continuous process.

First a solution of the polymer prepared in Example 1 g (i) was made by dissolving the polymer coating in water and then the plate and catheter were drawn through the solution of the polymer coating at a rate of 1 to 2 meters/second into an infrared dying oven of approximately 1 meter in length at about 100 °C. The objects were completely covered indicating the application of a substantially uniform coating. This process was done according to the standard procedures in US7442205, *Stents and methods for preparing stents from wires having hydrogel coating layers thereon.*

### Example 4

### Preparation of a coated working surface

The coatings 1 f (i) -(iv) and 2 c (i)-(iii) were spray coated onto an operating table. By means The operating table was spray coated with coating 2 c (i). Briefly, the method comprised the following steps:
(a) grounding the surface of the medical device that is to be coated
(b) applying a coating 2 c (i), which further comprised a chloroform as a solvent, by (1) providing the nozzle apparatus comprising a chamber connected to at least one opening for dispensing the coating 2 c (i); (2) placing the coating into the chamber; (3) electrically charging the coating formulation; (4) creating droplets of the electrically charged coating formulation; and (5) depositing the droplets of coating formulation onto the grounded surface to form a coating on the surface.

### Example 5

### Culturing of S. aureus

A clinical isolate of *Staphylococcus aureus* was obtained with consent from a patient admitted to a general surgery ward. A culture of the clinical isolate was made. A 10 mL suspension of the isolate was prepared with a cell density of 5x10⁸ cell/ml culture.

### Example 6

### Preparation of objects to be implanted

### a)Positive control

Orthopaedic plates, screws and a catheters as prepared in example 3 were incubated for 30 minutes in 10 mL of the isolate suspension as prepared in Example 5.

### b)Negative control

### i) Absence of S. aureus

An orthopaedic plate, two screws and a catheter were prepared according to Example 3. The coated objects were incubated in 10 mL of a solution of NaCl 0.9% (w/v) for 30 minutes. An overview of the prepared objects is shown in Table 4.

**Table 4**

| Example | | *S. aureus* | IRDye800 |
|---|---|---|---|
| 6 a | Orthopaedic plate | + | + |
| | Screw | + | + |
| | Catheter | + | + |
| 6 b | Orthopaedic plate | - | + |
| | Screw | - | + |
| | Catheter | - | + |

### Example 7

### Implantation of coated implants

Figures 6 and 8 show, respectively, photographs of an orthopaedic plate with screws, and a catheter coated with a polymer coating according to the Example 6(a), that have been implanted in a human ankle. Figure 10 shows a photograph of an orthopaedic plate, screws and a catheter coated with a polymer coating according to the Example 6 (b) that have been implanted in a human ankle, after closing of the implant wound.

The procedure for implantation of the objects coated in Example 6 (a) and (b), was carried out as followed:
The orthopaedic plates, screws and catheters were implanted in the ankle of a human cadaver. Implantation in a cadaver was used to simulate implantation of the said objects in a living patient. Each incubated plate was attached to the fibula with the respectively incubated screws, according to standard surgery procedures.

Each incubated catheter was inserted subcutaneously on the lateral side of the foot.

### Example 8

### Determining the presence of infection on an orthopaedic implant

Imaging of the objects implanted according to example 6 was performed 24 hours after implantation using an intra-operative clinical multispectral fluorescence camera (T3-imaging system, SurgOptix BV, Groningen, The Netherlands) and the IVIS Spectrum (excitation: 710nm, emission: 800nm, acquisition time 5s, binning 4, F-stop 2, FOV 21.2).

The results obtained from the IVIS Spectrum and IVIS Lumina II were analyzed with Living Image 4.2. (Caliper LS, Hopkinton MA, USA). Optimal detection limits were set to the lowest signal at which positive signal was effortlessly discriminated from negative controls. Signal intensity was determined by drawing regions of interests (ROI's) and measuring average counts in these regions. The signal was corrected for background by subtracting the background signal from the signal of interest, referred to in the text as net counts. A strong near infrared signal of 1x10⁵ ± 4.2x10⁴ counts; p=0.007 was, attributed to IRDye 800CW.

As can be seen in Figure 7, the coated orthopaedic plate and screws emitted a detectable signal, distinct from the background signal, in the presence of S. *aureus.* (white region in Figure 7). Figure 9 shows an image of a subcutaneously implanted catheter when infected with *S aureus* taken using a multi fluorescence camera .

The signals recorded in Figures 7 and 9 are indicative for the presence of *S. aureus* on or near the implant. S. *aureus* provides Sortase A, which is a compound specific for the LPETG (SEQ NO:5) motif contained in the peptide linker in the polymer coating. As described above, the linker was cleaved by the Sortase A compound and the IRDye800 was therefore released from polymer coating. Subsequently irradiation of the released IRDye800 by an infrared light induces photon emission from the dye which was recorded by the detector and can be seen in Figure 9 as a white spot.

Figure 11 shows an image of the control orthopaedic plate, screw and catheter prepared according to Example 6 (b), taken on a multispectral fluorescence camera. There is no intense white spot visible in the image, thus the coating does not emit a detectable signal indicating that the IRDye800 is still bound to the polymer coating and no *S. aureus* is therefore present.

### Example 9

### Detecting the presence of microbes on an implanted object

An implant comprising a polymer coating made from polymer conjugate 1 e (iv) prepared according to example 3, was implanted in a hind limb of immunocompetent mice (three in total). Three control mice received implants covered with the polymer conjugate 1 e (ii) that lacks the near infrared dye.

The six mice were subsequently injected with an inoculum of *S. aureus* strain Xen29, a standard, engineered strain of *S. aureus* that produces luciferase that facilitates localization of live bacteria by simultaneous imaging of luciferase bioluminescence and the fluorescence signal derived from IRDye800-vancomycin that is released from the polymer coating in the presence of *S. aureus.*

Three days after inoculation with *S. aureus* (Xen29), both bioluminescence and fluorescence imaging were performed with an IVIS Lumina II imaging system. A strong near-infrared fluorescence (NIRF) signal, attributed to IRDye800-vancomycin, appeared to co-localize with the bioluminescence signal (i.e. an intensity of 3.7x105 ± 9.8x104 counts). No NIRF signal was observed in infected mice that received an implant coated with polymer lacking the IRDye800.

Removal of tissue surrounded the implant and visualising the tissue using a flluoresence detector yielded a target to background ratio of 4.2 for the implant coated with 1 e (iv) corrected for background signal (thus seen as a white spot as described above), over the control implant (coated with 1 e (ii)).

### Example 10

### Discrimination of the presence of microbes from a sterile foreign body reaction

To discriminate a microbial infection from a sterile foreign body reaction or aseptic inflammation, the fluorescence signal detected after intravenous injection with *S. aureus* (Xen29)-induced myositis (that is an immune response due to an infection by *S. aureus,* n=6) was compared to that in mice with sterile myositis induced by sterilely implanted Cytodex beads (n=6) (that is an immune response due to a foreign body and **not** an infection).

Three of the mice injected with *S. aureus* (Xen29) had myositis in the left hind limb, as well as contralateral sterile myositis in order to reliably compare signals within the same animal. Ex vivo, the muscle tissue with sterile inflammation showed fluorescent signals (1.8x102 ± 0.8 x102 counts) comparable to those of healthy tissue (1.2x102 ± 0.1x102 counts, p=0.06, not significant). Again, muscle tissue infected with *S. aureus* (Xen29) emitted significantly higher fluorescence signals (4.1x102 ± 2.6x102 counts) in comparison to non-infected contralateral healthy muscle tissue (p=0.008) and tissue with sterile inflammation (p=0.01). This example shows that the IRDye800 is only released in the presence of a compound provided by *S. aureus* and thus release of the IRDye from the polymer coating is indicative for the presence of *S. aureus.* The polymer coating prepared according to the invention can therefore be used to indicate the presence of microbes on or near the surface of the implanted object. The presence of sterile inflammation following bead implantation was confirmed by light microscopy using Giemsa staining, i.e., accumulation of leukocytes around the beads and absence of leukocytes in healthy muscle tissue. Leukocytes were also detected by anti-CD45 pan leukocyte fluorescence staining (data not shown). No bioluminescent bacteria were found in cultures from either the non-infected or sterilely inflamed muscle tissue.

## Claims

1. Object comprising a polymer coating, the coating comprising:
a) one or more polymers, said polymers comprising a first cleavage site and
b) a first agent releasable from said coating upon cleavage of said first cleavage site **characterized in that** the first agent is covalently bound to a first polymer comprising polyethylene glycol via a first linker, the first linker comprising the first cleavage site being a peptide, wherein
the first cleavage site is cleaved by a sortase enzyme specifically provided by a microbe belonging to a first group consisting of Gram positive bacteria, and not cleaved by any compound provided by any microbe not belonging to said first group,
wherein cleavage of the said first cleavage site results in release of the said first agent from the coating, the release of said first agent being indicative for the presence of a microbe belonging to the said first group
wherein the first linker comprises a peptide.
wherein the first agent is a diagnostic agent being a fluorescent agent, wherein the object is a medical implant.

2. Object according to any of the preceding claims, wherein the first cleavage site comprises a specific amino acid motif, preferably being chosen from the group, consisting of LPATG (SEQ NO: 4), LPETG (SEQ NO: 5), LPDTG (SEQ NO: 6), LPQTG (SEQ NO: 7), NPQTN (SEQ NO: 8), NPKTN (SEQ NO: 9).
7.

3. Object according to any of the preceding claims wherein the fluorescent agent preferably is IRDye®800CW.

4. Object according to any of the preceding claims, wherein the coating comprises a first additional agent, which is released upon cleavage of the first cleavage site.

5. Object according to claim 4, the first additional agent is a therapeutic agent.

6. Object according to claim 4 or 5 wherein the first additional agent is covalently bound to a first additional polymer via a first additional linker, the first additional linker comprising the first cleavage site, wherein the first additional linker is identical to the first linker, wherein the first additional polymer is identical to the first polymer.

7. Object according to claims 4-6, wherein a polymer molecule of the first polymer comprises, covalently attached thereto, at least one molecule of the first agent and at least one molecule of the first additional agent.

8. Object according to claim 5, wherein the first additional agent is covalently attached to the at least first agent.

9. Object according to any of the claims -5-8, wherein at least one therapeutic agent is chosen from the group, consisting of beta-lactam antibiotics, cephalosporin antibiotics, macrolides, cyclic depsipeptides and tetracyclines, preferably being vancomycin.

10. Object according to any one of the preceding claims, wherein the first polymer, and/or, if present, the first additional polymer comprises a hydrogel.

11. Object according to any of the preceding claims, wherein the first polymer, and/or, if present, the first additional polymer comprises a biodegradable polymer.

12. Object according to any of the preceding claims, wherein the first polymer, and/or, if present, the first additional polymer comprises preferably polyethylene glycol vinyl-sulfone, more preferably polyethylene glycol diacrylate.

13. Object according to any of the preceding claims, the object being intended to be implanted in a patient's body during invasive surgery.

14. Method of producing a coated object according to anyone of claims 1-11, comprising the steps of:
a) dip coating the object in a solution of a polymer comprising comprising a first cleavage site and a first agent releasable from said coating upon cleavage of said first cleavage site wherein the first agent is covalently bound to a first polymer comprising polyethylene glycol via a first linker, the first linker comprising the first cleavage site being a peptide
wherein the first cleavage site is cleaved by a sortase enzyme specifically provided by a microbe belonging to a first group consisting of Gram positive bacteria, and not cleaved by any compound provided by any microbe not belonging to said first group, wherein cleavage of the said first cleavage site results in release of the said first agent from the coating, the release of said first agent being indicative for the presence of a microbe belonging to the said first group, and
c) drying the dipped object.

15. A polymer coating, the coating comprising
a) one or more polymers, said polymers comprising a first cleavage site and
b) a first agent releasable from said coating upon cleavage of said first cleavage site, **characterized in that** the first agent is covalently bound to a first polymer comprising polyethylene glycol via a first linker, the first linker comprising the first cleavage site being a peptide,
wherein the first cleavage site is cleaved by a sortase enzyme specifically provided by a microbe belonging to a first group consisting of Gram positive bacteria, and not cleaved by any compound provided by any microbe not belonging to said first group, wherein cleavage of the said first cleavage site results in release of the said first agent from the coating, wherein the first agent is a diagnostic agent being a fluorescent agent.

## Patentansprüche

1. Gegenstand, umfassend eine Polymerbeschichtung, wobei die Beschichtung umfasst:
a) ein oder mehrere Polymere, wobei die Polymere eine erste Spaltungsstelle umfassen, und
b) ein erstes Mittel, freisetzbar aus der Beschichtung nach Spaltung der ersten Spaltungsstelle, **dadurch gekennzeichnet, dass** das erste Mittel kovalent gebunden an ein erstes Polymer, umfassend Polyethylenglykol, über einen ersten Linker ist, wobei der erste Linker die erste Spaltungsstelle, die ein Peptid ist, umfasst,
wobei die erste Spaltungsstelle gespalten wird durch ein Sortase-Enzym, das speziell bereitgestellt wird von einer Mikrobe, die zu einer aus grampositiven Bakterien bestehenden ersten Gruppe gehört, und nicht gespalten wird durch eine Verbindung, die bereitgestellt wird von einer nicht zu der ersten Gruppe gehörenden Mikrobe,
wobei die Spaltung der ersten Spaltungsstelle zur Freisetzung des ersten Mittels aus der Beschichtung führt, wobei die Freisetzung des ersten Mittels indikativ für das Vorhandensein einer zu der ersten Gruppe gehörenden Mikrobe ist,
wobei der erste Linker ein Peptid umfasst,
wobei das erste Mittel ein Diagnostikum ist, das ein fluoreszierendes Mittel ist,
wobei der Gegenstand ein medizinisches Implantat ist.

2. Gegenstand nach einem der vorangehenden Ansprüche, wobei die erste Spaltungsstelle ein spezifisches Aminosäuremotiv umfasst, das vorzugsweise ausgewählt ist aus der Gruppe, bestehend aus LPATG (SEQ NO: 4), LPETG (SEQ NO: 5), LPDTG (SEQ NO: 6), LPQTG (SEQ NO: 7), NPQTN (SEQ NO: 8), NPKTN (SEQ NO: 9).

3. Gegenstand nach einem der vorangehenden Ansprüche, wobei das fluoreszierende Mittel vorzugsweise IRDye® 800CW ist.

4. Gegenstand nach einem der vorangehenden Ansprüche, wobei die Beschichtung ein erstes zusätzliches Mittel umfasst, das nach Spaltung der ersten Spaltungsstelle freigesetzt wird.

5. Gegenstand nach Anspruch 4, wobei das erste zusätzliche Mittel ein Therapeutikum ist.

6. Gegenstand nach Anspruch 4 oder 5, wobei das erste zusätzliche Mittel kovalent gebunden an ein erstes zusätzliches Polymer über einen ersten zusätzlichen Linker ist, wobei der erste zusätzliche Linker die erste Spaltungsstelle umfasst, wobei der erste zusätzliche Linker identisch mit dem ersten Linker ist, wobei das erste zusätzliche Polymer identisch mit dem ersten Polymer ist.

7. Gegenstand nach den Ansprüchen 4-6, wobei ein Polymermolekül des ersten Polymers, kovalent daran gebunden, mindestens ein Molekül des ersten Mittels und mindestens ein Molekül des ersten zusätzlichen Mittels umfasst.

8. Gegenstand nach Anspruch 5, wobei das erste zusätzliche Mittel kovalent gebunden an das mindestens erste Mittel ist.

9. Gegenstand nach einem der Ansprüche 5-8, wobei mindestens ein Therapeutikum ausgewählt ist aus der Gruppe, bestehend aus β-Lactam-Antibiotika, Cephalosporin-Antibiotika, Makroliden, cyclischen Depsipeptiden und Tetracyclinen, vorzugsweise Vancomycin ist.

10. Gegenstand nach einem der vorangehenden Ansprüche, wobei das erste Polymer und/oder, wenn vorhanden, das erste zusätzliche Polymer ein Hydrogel umfasst.

11. Gegenstand nach einem der vorangehenden Ansprüche, wobei das erste Polymer und/oder, wenn vorhanden, das erste zusätzliche Polymer ein biologisch abbaubares Polymer umfasst.

12. Gegenstand nach einem der vorangehenden Ansprüche, wobei das erste Polymer und/oder, wenn vorhanden, das erste zusätzliche Polymer vorzugsweise Polyethylenglykolvinylsulfon, stärker bevorzugt Polyethylenglykoldiacrylat.

13. Gegenstand nach einem der vorangehenden Ansprüche, wobei der Gegenstand dazu vorgesehen ist, in einen Körper eines Patienten während einer invasiven Operation implantiert zu werden.

14. Verfahren zur Herstellung eines beschichteten Gegenstandes nach einem der Ansprüche 1-11, umfassend die folgenden Schritte:
a) Tauchbeschichten des Gegenstandes in einer Lösung eines Polymers, umfassend eine erste Spaltungsstelle, und
b) ein erstes Mittel, freisetzbar aus der Beschichtung nach Spaltung der ersten Spaltungsstelle, wobei das erste Mittel kovalent gebunden an ein erstes Polymer, umfassend Polyethylenglykol, über einen ersten Linker ist, wobei der erste Linker die erste Spaltungsstelle, die ein Peptid ist, umfasst,
wobei die erste Spaltungsstelle gespalten wird durch ein Sortase-Enzym, das speziell bereitgestellt wird von einer Mikrobe, die zu einer aus grampositiven Bakterien bestehenden ersten Gruppe gehört, und nicht gespalten wird durch eine Verbindung, die bereitgestellt wird von einer nicht zu der ersten Gruppe gehörenden Mikrobe, wobei die Spaltung der ersten Spaltungsstelle zur Freisetzung des ersten Mittels aus der Beschichtung führt, wobei die Freisetzung des ersten Mittels indikativ für das Vorhandensein einer zu der ersten Gruppe gehörenden Mikrobe ist, wobei das erste Mittel ein Diagnostikum ist, das ein fluoreszierendes Mittel ist, und
c) Trocknen des getauchten Gegenstandes.

15. Polymerbeschichtung, wobei die Beschichtung umfasst:
a) ein oder mehrere Polymere, wobei die Polymere eine erste Spaltungsstelle umfassen, und
b) ein erstes Mittel, freisetzbar aus der Beschichtung nach Spaltung der ersten Spaltungsstelle, **dadurch gekennzeichnet, dass** das erste Mittel kovalent gebunden an ein erstes Polymer, umfassend Polyethylenglykol, über einen ersten Linker ist, wobei der erste Linker die erste Spaltungsstelle, die ein Peptid ist, umfasst,
wobei die erste Spaltungsstelle gespalten wird durch ein Sortase-Enzym, das speziell bereitgestellt wird von einer Mikrobe, die zu einer aus grampositiven Bakterien bestehenden ersten Gruppe gehört, und nicht gespalten wird durch eine Verbindung, die bereitgestellt wird von einer nicht zu der ersten Gruppe gehörenden Mikrobe, wobei die Spaltung der ersten Spaltungsstelle zur Freisetzung des ersten Mittels aus der Beschichtung führt, wobei das erste Mittel ein Diagnostikum ist, das ein fluoreszierendes Mittel ist.

## Revendications

1. Objet comprenant un revêtement polymère, le revêtement comprenant :
a) un ou plusieurs polymères, lesdits polymères comprenant un premier site de clivage et
b) un premier agent pouvant être libéré dudit revêtement lors du clivage dudit premier site de clivage, **caractérisé en ce que** le premier agent est lié par covalence à un premier polymère comprenant du polyéthylène glycol via un premier agent de liaison, le premier agent de liaison comprenant le premier site de clivage étant un peptide,
dans lequel le premier site de clivage est clivé par une enzyme sortase spécifiquement fournie par un microbe appartenant à un premier groupe constitué de bactéries à Gram positif, et est non clivé par un quelconque composé fourni par un microbe n'appartenant pas audit premier groupe,
dans lequel le clivage dudit premier site de clivage a pour résultat la libération dudit premier agent depuis le revêtement, la libération dudit premier agent étant indicative de la présence d'un microbe appartenant audit premier groupe,
dans lequel le premier agent de liaison comprend un peptide,
dans lequel le premier agent est un agent de diagnostic qui est un agent fluorescent,
dans lequel l'objet est un implant médical.

2. Objet selon l'une quelconque des revendications précédentes, dans lequel le premier site de clivage comprend un motif d'acides aminés spécifique, de préférence qui est choisi parmi le groupe, constitué de LPATG (SEQ NO : 4), LPETG (SEQ NO : 5), LPDTG (SEQ NO : 6), LPQTG (SEQ NO : 7), NPQTN (SEQ NO : 8), NPKTN (SEQ NO : 9).

3. Objet selon l'une quelconque des revendications précédentes, dans lequel l'agent fluorescent est de préférence IRDye®800CW.

4. Objet selon l'une quelconque des revendications précédentes, dans lequel le revêtement comprend un premier agent additionnel, qui est libéré lors du clivage du premier site de clivage.

5. Objet selon la revendication 4, dans lequel le premier agent additionnel est un agent thérapeutique.

6. Objet selon la revendication 4 ou 5 dans lequel le premier agent additionnel est lié par covalence à un premier polymère additionnel via un premier agent de liaison additionnel, le premier agent de liaison additionnel comprenant le premier site de clivage, dans lequel le premier agent de liaison additionnel est identique au premier agent de liaison, dans lequel le premier polymère additionnel est identique au premier polymère.

7. Objet selon les revendications 4-6, dans lequel une molécule polymère du premier polymère comprend, liées par covalence à celui-ci, au moins une molécule du premier agent et au moins une molécule du premier agent additionnel.

8. Objet selon la revendication 5, dans lequel le premier agent additionnel est lié par covalence au au moins premier agent.

9. Objet selon l'une quelconque des revendications 5 à 8, dans lequel au moins un agent thérapeutique est choisi parmi le groupe constitué de d'antibiotiques bêta-lactamines, antibiotiques céphalosporines, macrolides, depsipeptides cycliques et tétracyclines, de préférence qui est la vancomycine.

10. Objet selon l'une quelconque des revendications précédentes, dans lequel le premier polymère, et/ou, s'il est présent, le premier polymère additionnel comprennent un hydrogel.

11. Objet selon l'une quelconque des revendications précédentes, dans lequel le premier polymère, et/ou, s'il est présent, le premier polymère additionnel comprennent un polymère biodégradable.

12. Objet selon l'une quelconque des revendications précédentes, dans lequel le premier polymère, et/ou, s'il est présent, le premier polymère additionnel, comprennent de préférence du polyéthylèneglycol vinylsulfone, de manière plus préférée du polyéthylèneglycol diacrylate.

13. Objet selon l'une quelconque des revendications précédentes, l'objet étant destiné à être implanté dans le corps d'un patient lors d'une chirurgie invasive.

14. Méthode de production d'un objet revêtu selon l'une quelconque des revendications 1-11, comprenant les étapes consistant à :
a) revêtir par immersion l'objet dans une solution d'un polymère comprenant un premier site de clivage et
b) un premier agent pouvant être libéré dudit revêtement lors du clivage dudit premier site de clivage, le premier agent étant lié par covalence à un premier polymère comprenant du polyéthylène glycol via un premier agent de liaison, le premier agent de liaison comprenant le premier site de clivage étant un peptide,
dans lequel le premier site de clivage est clivé par une enzyme sortase spécifiquement fournie par un microbe appartenant à un premier groupe constitué de bactéries à Gram positif, et est non clivé par un quelconque composé fourni par un quelconque microbe n'appartenant pas audit premier groupe, ledit clivage dudit site de clivage ayant pour résultat la libération dudit premier agent depuis le revêtement, la libération dudit premier agent étant indicative de la présence d'un microbe appartenant audit premier groupe, dans lequel le premier agent est un agent de diagnostic qui est un agent fluorescent, et
b) sécher l'objet ayant été immergé.

15. Revêtement polymère, le revêtement comprenant
a) un ou plusieurs polymères, lesdits polymères comprenant un premier site de clivage et
b) un premier agent pouvant être libéré dudit revêtement lors du clivage dudit premier site de clivage, **caractérisé en ce que** le premier agent est lié par covalence à un premier polymère comprenant du polyéthylèneglycol via un premier agent de liaison, le premier agent de liaison comprenant le premier site de clivage étant un peptide,
dans lequel le premier site de clivage est clivé par une enzyme sortase spécifiquement fournie par un microbe appartenant à un premier groupe constitué de bactéries à Gram positif, et est non clivé par un quelconque composé fourni par un quelconque microbe n'appartenant pas audit premier groupe, ledit clivage dudit site de clivage ayant pour résultat la libération dudit premier agent depuis le revêtement, dans lequel le premier agent est un agent de diagnostic qui est un agent fluorescent.


### SEQUENCE LISTING

<110> INDIANA B.V.
<120> Cleavable coating material having microbial functionality
<130> 52638PC
<150> NL 2010040
   <151> 2012-12-21
<160> 17
<170> PatentIn version 3.5
<210> 1
   <211> 7
   <212> PRT
   <213> Artificial Sequence
<220>
   <223> Adhesive matrix molecule domain
<400> 1
   Met Ser Cys Arg Ala Met Met
   1               5
<210> 2
   <211> 4
   <212> PRT
   <213> Artificial Sequence
<220>
   <223> Cleavage site 1
<400> 2
   Pro Pro Thr Pro
   1
<210> 3
   <211> 4
   <212> PRT
   <213> Artificial Sequence
<220>
   <223> Cleavage site 2
<400> 3
   Pro Pro Ser Pro
   1
<210> 4
   <211> 5
   <212> PRT
   <213> Artificial Sequence
<220>
   <223> Cleavage site 3
<400> 4
   Leu Pro Ala Thr Gly
   1               5
<210> 5
   <211> 5
   <212> PRT
   <213> Artificial Sequence
<220>
   <223> Cleavage site 4
<400> 5
   Leu Pro Glu Thr Gly
   1               5
<210> 6
   <211> 5
   <212> PRT
   <213> Artificial Sequence
<220>
   <223> Cleavage site 5
<400> 6
   Leu Pro Asp Thr Gly
   1               5
<210> 7
   <211> 5
   <212> PRT
   <213> Artificial Sequence
<220>
   <223> Cleavage site 6
<400> 7
   Leu Pro Gln Thr Gly
   1               5
<210> 8
   <211> 5
   <212> PRT
   <213> Artificial Sequence
<220>
   <223> Cleavage site 7
<400> 8
   Leu Pro Gln Thr Gly
   1               5
<210> 9
   <211> 5
   <212> PRT
   <213> Artificial Sequence
<220>
   <223> Cleavage site 8
<400> 9
   Asn Pro Gln Thr Asn
   1 5
<210> 10
   <211> 5
   <212> PRT
   <213> Artificial Sequence
<220>
   <223> Cleavage site 9
<400> 10
   Asn Pro Lys Thr Asn
   1 5
<210> 11
   <211> 5
   <212> PRT
   <213> Artificial Sequence
<220>
   <223> Cleavage site 10
<400> 11
   Gly Phe Pro Arg Gly
   1 5
<210> 12
   <211> 5
   <212> PRT
   <213> Artificial Sequence
<220>
   <223> Cleavage site 11
<220>
   <221> MISC_FEATURE
   <222> (2)..(2)
   <223> 'D'amino acid
<400> 12
   Gly Phe Pro Arg Gly
   1 5
<210> 13
   <211> 5
   <212> PRT
   <213> Artificial Sequence
<220>
   <223> Sortase catalytic pocket
<220>
   <221> misc_feature
   <222> (3)..(3)
   <223> Xaa can be any naturally occurring amino acid
<400> 13
   Thr Leu Xaa Thr Cys
   1 5
<210> 14
   <211> 14
   <212> PRT
   <213> Artificial Sequence
<220>
   <223> Linker 1
<400> 14
   Gly Gly Gly Gly Phe Pro Arg Gly Phe Pro Ala Gly Gly Gly
   1 5 10
<210> 15
   <211> 12
   <212> PRT
   <213> Artificial Sequence
<220>
   <223> Linker 2
<220>
   <221> MISC_FEATURE
   <222> (2)..(2)
   <223> D amino acid
<400> 15
   Gly Phe Pro Arg Gly Phe Pro Ala Gly Gly Glu Gly
   1 5 10
<210> 16
   <211> 14
   <212> PRT
   <213> Artificial Sequence
<220>
   <223> Linker having LPETG cleavage site
<400> 16
   Cys Gly Gly Gly Leu Pro Glu Thr Gly Gly Ser Gly Gly Lys
   1 5 10
<210> 17
   <211> 13
   <212> PRT
   <213> Artificial Sequence
<220>
   <223> Linker having AAF cleavage site
<400> 17
   Cys Gly Gly Gly Ala Ala Phe Gly Gly Ser Gly Gly Lys
   1               5                   10